# EUROPEAN PATENT APPLICATION

(11) **EP 2 716 662 A1**
(43) Date of publication of application: **09.04.2014**
(21) Application number: 12792145.0
(22) Date of filing: 30.05.2012
(51) Int. Cl.: C08B 37/08, C08B 15/10, C08B 37/02, C08K 5/3472, C08L 5/00

(54) **HYDROGEL AND METHOD FOR PRODUCING SAME**

(30) Priority: 31.05.2011 JP 2011122183
(71) Applicant: The University of Tokyo, Bunkyo-Ku Tokyo 113-8654 (JP)
(72) Inventor: ITO Taichi, Tokyo 113-8654 (JP); SUZUKI Yukimitsu, Tokyo 113-8654 (JP); TAKAHASHI Akira, Tokyo 113-8654 (JP); SHIMIZU Atsushi, Tokyo 113-8654 (JP)
(74) Representative: Hirsch & Associés
(86) International application number: PCT/JP2012/063892
(87) International publication number: WO 2012/165462

(57) **Abstract**

[Problem] To provide a hydrogel which is obtained by reacting an azide group and a cyclooctyne group in the absence of a catalyst, especially in the absence of a copper catalyst; in particular, to provide a hydrogel which can be used for a biocompatible material.

[Solution] The above-mentioned problem can be solved by a hydrogel which has: (a) a first polymer moiety composed of hyaluronic acid, carboxymethyl dextran or the like; (b) a second polymer moiety composed of hyaluronic acid, carboxymethyl dextran or the like, said second polymer moiety being of a kind that is same as or different from the kind of the moiety (a) and is composed of a molecule different from the moiety (a); and (c) a triazole ring group or a derivative group thereof. The hydrogel has a structure wherein the first polymer moiety (a) and the second polymer moiety (b) are crosslinked with each other via the triazole ring group or a derivative group thereof (c).

## Description

### TECHNICAL FIELD

The present invention relates to a hydrogel and a method of producing same, as well as a feedstock for the hydrogel and a method of producing the feedstock.

### BACKGROUND ART

*In situ* crosslinking gels can be used for medical devices such as, for example, adhesion-preventing materials, regenerative therapy scaffolds, and/or drug delivery system carriers, and multiple studies thereof have been performed.

Polysaccharides, especially hyaluronic acid, exhibit superior biocompatibility, leading to their wide uses in ophthalmic solutions, palliatives for arthralgia, abdominal adhesion barriers, and the like. Non-patent reference 1 discloses, in detail, a hydrogel using hyaluronic acid. In particular, non-patent reference 1 comprehensively discloses a method of forming covalent bonds to prepare a hydrogel. However, due to the problematic possibility of cytotoxicity due to nonspecific reactions with biomolecules presented by this method of forming covalent bonds, its use in *in vivo* applications is limited.

Meanwhile, patent reference 1 discloses a hydrogel obtained using a click reaction. In particular, patent reference 1 discloses a hydrogel using a peptide and polyethylene glycol as a skeleton. However, the polyethylene glycol used in patent reference 1 does not breakdown within the body, and peptides often exhibit antigenicity, posing difficulties to their use in *in vivo* environments. In actuality, patent reference 1 contains no disclosure whatsoever of using a click reaction for an *in situ* crosslinking gel.

### PRIOR ART REFERENCES

### PATENT REFERENCES

Patent Reference 1: WO/2009/039307

### NON-PATENT REFERENCES

Non-patent Reference 1: ADVANCED MATERIALS Vol. 23, 12, pp. H41-H56 (2011)

### SUMMARY OF THE INVENTION

### PROBLEMS TO BE SOLVED BY THE INVENTION

Thus, an object of the present invention is to provide a novel hydrogel, particularly a novel hydrogel using a polysaccharide as polymer skeleton, and more particularly to a novel *in situ* crosslinking hydrogel exhibiting biocompatibility in *in vivo* environments.

Apart from this object, another object of the present invention, in addition to the first object, is to provide a material using the hydrogel, particularly a biocompatible material exhibiting superior biodegradability.

Apart from these objects, a further object of the present invention, in addition to the abovementioned objects, is to provide a method of producing the hydrogel.

Apart from these objects, a further object of the present invention, in addition to the abovementioned objects, is to provide a chemical agent constituting a feedstock for the hydrogel, and a method of producing the same.

### MEANS USED TO SOLVE THE ABOVE-MENTIONED PROBLEMS

The inventors discovered the following inventions.
<1> A hydrogel comprising:
   a) a first polymer section selected from the group consisting of hyaluronic acid, carboxymethyl dextran, cellulose derivatives (e.g., carboxymethyl cellulose), and chitosan;
   b) a second polymer section, selected from the group consisting of hyaluronic acid, carboxymethyl dextran, cellulose derivatives (e.g., carboxymethyl cellulose), and chitosan, that may be the same or different from a), but comprising a different molecule; and
   c) a triazole ring group or derivative group thereof;
      the hydrogel having a structure such that the a) first polymer section and b) second polymer section are crosslinked via the mediation of the c) triazole ring group or derivative group thereof.
<2> In <1> described above, there is a spacer group X1 (X1 representing a single bond or a group having a molecular weight of 10,000 or less) between the a) first polymer section and the c) triazole ring group or derivative group thereof. X1 is preferably an alkylene group or polysaccharide-derived group comprising at least one substituent selected from the group consisting of an ether group, an ester group, an amide group, a hydrazide group, a disulfide group, and a carbonyl group, more preferably an alkylene group or polysaccharide-derived group comprising an ester group, an ether group, or an amide group.
<3> In <1> or <2>, there is a spacer group X2 (X2 representing a single bond or group having a molecular weight of 10,000 or less) between the second polymer section and the c) triazole ring group or derivative group thereof. X2 is an alkylene group or polysaccharide-derived group comprising at least one substituent selected from the group consisting of an ether group, an ester group, an amide group, a hydrazide group, a disulfide group, and a carbonyl group, preferably an alkylene group or polysaccharide-derived group comprising an ester group, an ether group, or an amide group.
<4> In <2> or <3>, X1 is substituted for an -OH group of the a-1) first polymer section to form a bond, thereby forming -O-X1-, preferably -O-CO-X11-, -CO-NH-X11-, -CO-NH-NH-X11-, -S-S-X11-, or -CO-X11- (X11 being defined similarly to X1 described above), more preferably -O-CO-X11- or -CO-NH-X11-. O-CO-(CH₂)₃- or -CO-NH-(CH₂CH₂O)ₘ-NH-CO-(CH₂)₃- (m representing an integer of 1 or greater) is more preferable. Alternatively, if the a-2) first polymer section comprises a -COOY group (Y signifying a monovalent cation of hydrogen, sodium, or the like), the group is substituted to form a bond, thereby forming -CO-X1-, preferably -CO-NH-X11-, -CO-NH-NH-X11-, or -CO-O-X11- (defined similarly to X1 above), more preferably -CO-O-X11- or -CO-NH-X11-. If the a-3) first polymer section comprises a -NH₂ group, the group is substituted to form a bond, thereby forming -NH-X1-, preferably -NH-CO-X11- or -NH-CO-NH-X11- (X11 being defined similarly to X1 above).
<5> In either of <3> or <4>, X2 is substituted for an -OH group of the b-1) second polymer section to form a bond, thereby forming -O-X2-, preferably -O-CO-X21-, -CO-NH-X21-, -CO-NH-NH-X21-, -S-S-X21-, or -CO-X21- (X21 being defined similarly to X2 above), more preferably -O-CO-X21- or -CO-NH-X21. -CO-CH₂-O- or -CO-NH- (CH₂CH₂O)ₘ-NH-CO-CH₂-O- (m representing an integer of 1 or greater) is more preferable. Alternatively, if the b-2) second polymer section comprises a -COOY group (Y signifying a monovalent cation of hydrogen, sodium, or the like), the group is substituted to form a bond, thereby forming -COX2-, preferably -CO-NH-X21-, -CO-NH-NH-X21-, or -CO-OX21- (X21 being defined similarly to X1 above), more preferably -CO-O-X21- or -CO-NH-X21-. If the b-3) first polymer section comprises a -NH₂ group, the group is substituted to form a bond, thereby forming -NH-X2-, preferably -NH-CO-X21- or -NH-CO-NH-X21- (X21 being defined similarly to X2 above).
<6> In any of <1> through <5>, the c) triazole ring group or derivative group thereof is present in an amount equivalent to 5-60 mol%, preferably 10-50 mol%, more preferably 20-30 mol%, taking the total amount of - OH groups or total amount of -NH₂ groups in the a) first polymer section as 100 mol%.
<7> In any of <1> through <6>, the c) triazole ring group or derivative group thereof is present in an amount equivalent to 5-60 mol%, preferably 10-50 mol%, more preferably 20-30 mol%, taking the total amount of -OH groups or total amount of -NH₂ groups in the b) second polymer section as 100 mol%.
<8> In any of <2> through <7>, -X1- is -CO-(CH₂)₃-or -CO-NH-(CH₂CH₂O)ₘ-NH-CO-(CH₂)₃- (m representing an integer of 1 or greater).
<9> In any of <3> through <8>, -X2- is -CO-CH₂-O- or -CO-NH-(CH₂CH₂O)ₘ-NH-CO-CH₂-O- (m representing an integer of 1 or greater).
<10> In any of <1> through <9>, the a) first polymer section and b) second polymer section are hyaluronic acid.
<11> In any of <1> through <9>, the a) first polymer section and b) second polymer section are carboxymethyl dextran.
<12> In any of <1> through <9>, the a) first polymer section and b) second polymer section are chitosan.
<13> A biocompatible material comprising the hydrogel according to any of <1> through <12>.
<14> In <13>, the biocompatible material is an adhesion barrier.
<15> In <13>, the biocompatible material is a drug delivery system carrier.
<16> In <13>, the biocompatible material is a cell-encapsulating material.
<17> A kit for creating the hydrogel according to any of <1> through <12>.
<18> A method of producing a hydrogel comprising the steps of:
   A) readying a liquid containing a) a first polymer selected from the group consisting of hyaluronic acid, carboxymethyl dextran, cellulose derivatives (e.g., carboxymethyl cellulose), and chitosan, the first polymer comprising an azide group;
   B) readying a liquid comprising b) a second polymer, selected from the group consisting of hyaluronic acid, carboxymethyl dextran, cellulose derivatives (e.g., carboxymethyl cellulose), and chitosan, that may be the same as or different from a), but comprising a different molecule than a), the second polymer comprising a cyclooctyne group or a cyclooctyne group derivative; and
   C) mixing a) the first liquid and b) the second liquid;
      thereby inducing a click reaction between the azide group and the cyclooctyne group or cyclooctyne group derivative, thus forming a triazole ring or derivative thereof, and forming a hydrogel having a structure in which the first polymer and the second polymer are crosslinked via the mediation of the triazole ring or derivative thereof.
<19> In <18>, it is preferable that, in the mixing step C), one half of a double syringe be filled with the liquid comprising the a) first polymer and the other half with the liquid containing the b) second polymer, and the liquid containing the a) first polymer and the liquid containing the b) second polymer be dispensed from the double syringe roughly simultaneously. It is preferable that the mixing step C) be performed *in vivo,* as indicated in example 18. In the production method according to <18>, the kit according to <17> can also be used to prepare the hydrogel.
<20> In <18> or <19>, there is a spacer group X1 (X1 representing a single bond or a group having a molecular weight of 10,000 or less) between the a) first polymer and the azide group. X1 is an alkylene group or polysaccharide-derived group comprising at least one substituent selected from the group consisting of an ether group, an ester group, an amide group, a hydrazide group, a disulfide group, and a carbonyl group, preferably an alkylene group or polysaccharide-derived group comprising an ester group, an ether group, or an amide group.
<21> In any of <18> through <20>, there is a spacer group X2 (X2 representing a single bond or a group having a molecular weight of 10,000 or less) between the b) second polymer and the cyclooctyne group or cyclooctyne group derivative. X2 is an alkylene group or polysaccharide-derived group comprising at least one substituent selected from the group consisting of an ether group, an ester group, an amide group, a hydrazide group, a disulfide group, and a carbonyl group, preferably an alkylene group or polysaccharide-derived group comprising an ester group, an ether group, or an amide group.
<22> In any of <18> through <21>, X1 is substituted for an -OH group of the a-1) first polymer to form a bond, thereby forming -O-X1-, preferably -O-COX11-, -CO-NH-X11-, -CO-NH-NH-X11-, -S-S-X11-, or -CO-X11-(X11 representing an alkylene group or polysaccharide-derived group similar to that of X1 described above), more preferably -O-CO-X11- or -CO-NH-X11-. Alternatively, if the a-2) first polymer section comprises a -COOY group (Y signifying a monovalent cation of hydrogen, sodium, or the like), the group is substituted to form a bond, thereby forming -CO-X1-, preferably -CO-NH-X11-, -CO-NH-NH-X11-, or -CO-O-X11- (X11 representing an alkylene group or polysaccharide-derived group similar to that of X1 above), more preferably -CO-O-X11- or -CO-NH-X11-. If the a-3) first polymer section comprises a -NH₂ group, the group is substituted to form a bond, thereby forming -NH-X1-, preferably -NH-CO-X11- or -NH-CO-NH-X11- (X11 being defined similarly to X1 above).
<23> In any of <18> through <22>, X2 is substituted for an -OH group of the b-1) second polymer, forming -O-X2-, preferably -O-CO-X21-, -CO-NH-X21-, -CO-NH-NH-X21-, -S-S-X21-, or -CO-X21- (X21 representing an alkylene group or polysaccharide-derived group similar to that of X2 above), more preferably -O-CO-X21- or -CO-NH-X21-. Alternatively, if the b-2) second polymer section comprises a -COOY group (Y signifying a monovalent cation of hydrogen, sodium, or the like), the group is substituted to form a bond, thereby forming -CO-X2-, preferably -CO-NH-X21-, -CO-NH-NH-X21-, or -CO-O-X21-(X21 representing an alkylene group or polysaccharide-derived group similar to that of X2 above), more preferably -CO-O-X21- or -CO-NH-X21-. If the b-3) second polymer section comprises a -NH₂ group, the group is substituted to form a bond, thereby forming -NH-X2-, preferably -NH-CO-X21- or -NH-CO-NH-X21- (X21 being defined similarly to X2 above).
<24> In any of <18> through <23>, the azide group is present in an amount equivalent to 5-60 mol%, preferably 10-50 mol%, more preferably 20-30 mol%, taking the total amount of -OH groups or total amount of -NH₂ groups in the a) first polymer as 100 mol%.
<25> In any of <18> through <24>, the cyclooctyne group or cyclooctyne group derivative is present in an amount equivalent to 5-60 mol%, preferably 10-50 mol%, more preferably 20-30 mol%, taking the total amount of - OH groups or total amount of -NH₂ groups in the b) second polymer as 100 mol%.
<26> In any of <20> through <25>, -X1- is -CO-(CH₂)₃- or -CO-NH-(CH₂CH₂O)ₘ-NH-CO-(CH₂)₃- (m representing an integer of 1 or greater)
<27> In any of <21> through <26>, -X2- is -CO-CH₂-O- or -CO-NH-(CH₂CH₂O)ₘ-NH-CO-CH₂-O- (m representing an integer of 1 or greater).
<28> In any of <18> through <27>, the a) first polymer and b) second polymer are hyaluronic acid.
<29> In any of <18> through <27>, the a) first polymer and b) second polymer are carboxymethyl dextran.
<30> In any of <18> through <27>, the a) first polymer and b) second polymer are chitosan.
<31> Hyaluronic acid whose -OH groups have been at least partially substituted by a -O-X3-N₃ group, X3 being an alkylene group comprising at least one substituent selected from the group consisting of an ether group, an ester group, an amide group, a hydrazide group, a disulfide group, and a carbonyl group. X3 is an alkylene group or polysaccharide-derived group comprising an ether group or an ester group in addition to -O-. The -O-X3-N₃ group forms -O-CO-X31- or -O-X31- (X31 representing an alkylene group-comprising group or polysaccharide-derived group similar to X3 above), preferably -O-CO-X31-.
<32> In <31>, -X3- is -CO-(CH₂)₃- or -CO-NH-(CH₂CH₂O)ₘ-NH-CO-(CH₂)₃- (m representing an integer of 1 or greater).
<33> In <31> or <32>, the hyaluronic acid has the following formula (1) (in which at least one R is a group represented by formula (1)-1, the rest represent H, and n represents an integer from 100 to 20,000. 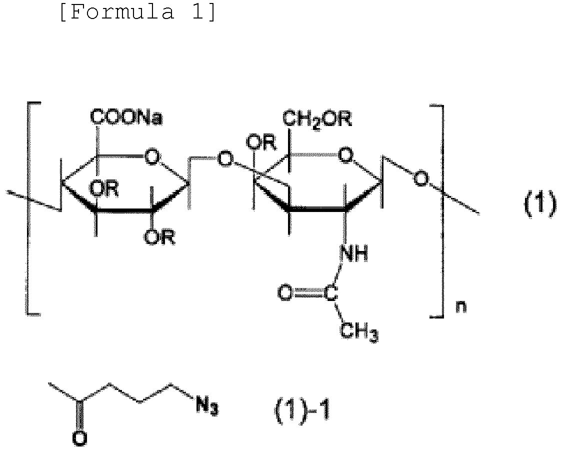
<34> Hyaluronic acid whose -OH groups have been at least partially substituted by a -O-X4-(cyclooctyne or cyclooctyne derivative) group, X4 being an alkylene group comprising at least one substituent selected from the group consisting of an ether group, an ester group, an amide group, a hydrazide group, a disulfide group, and a carbonyl group. X4 is an alkylene group or polysaccharide-derived group comprising an ether group or an ester group in addition to -O-. The -O-X4-(cyclooctyne or cyclooctyne derivative) group preferably forms -O-CO-X41- or -O-X41- (X41 being an alkylene group-comprising group or polysaccharide-derived group similar to X4), more preferably -O-CO-X41-.
<35> In <34>, -X2- is -CO-CH₂-O- or -CO-NH-(CH₂CH₂O)ₘ-NH-CO-CH₂-O- (m representing an integer of 1 or greater).
<36> In <34> or <35>, the hyaluronic acid has the following formula (2) (in which at least one R is a group represented by formula (2)-1), the rest represent H, and n' represents an integer from 100 to 20,000). 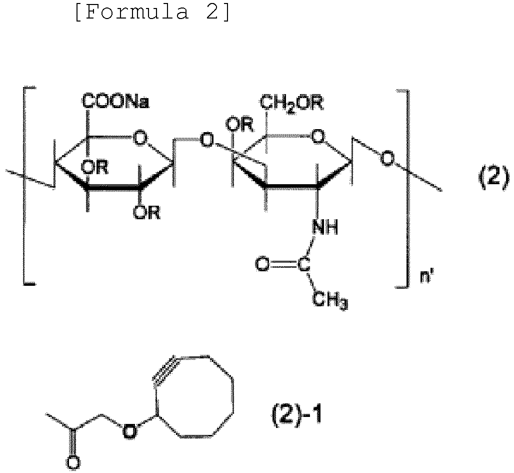
<37> Carboxymethyl dextran whose -OH groups have been at least partially substituted by a -O-X3-N₃ group, X3 being an alkylene group comprising at least one substituent selected from the group consisting of an ether group, an ester group, an amide group, a hydrazide group, a disulfide group, and a carbonyl group. X3 is an alkylene group or polysaccharide-derived group comprising an ether group or an ester group in addition to -O-. The -O-X3-N₃ group forms -O-CO-X31- or -O-X31- (X31 representing an alkylene group-comprising group or polysaccharide-derived group similar to X3 above), preferably -O-CO-X31-.
<38> In <37>, -X3- is -CO-(CH₂)₃- or -CO-NH-(CH₂CH₂O)ₘ-NH-CO-(CH₂)₃- (m representing an integer of 1 or greater).
<39> In <37> or <38>, the carboxymethyl dextran has the following formula (3) (in which one *R*₁ is CH₂COONa, at least one is a group represented by formula (3)-1, the rest are H, and i is an integer from 100 to 20,000). 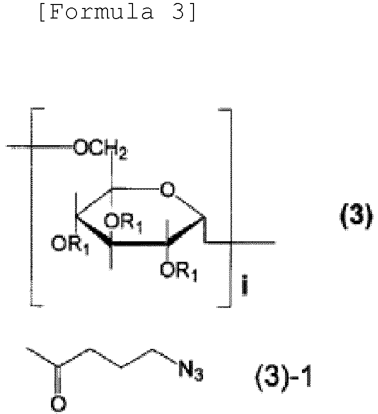
<40> Carboxymethyl dextran whose -OH groups have been at least partially substituted by a -O-X4-(cyclooctyne or cyclooctyne derivative) group, X4 being an alkylene group comprising at least one substituent selected from the group consisting of an ether group, an ester group, an amide group, a hydrazide group, a disulfide group, and a carbonyl group. X4 is an alkylene group or polysaccharide-derived group comprising an ether group or an ester group in addition to -O-. The -O-X4-(cyclooctyne or cyclooctyne derivative) group forms -O-CO-X41- or -O-X41- (X41 being an alkylene group-comprising group or polysaccharide-derived group similar to X4), preferably -O-CO-X41-.
<41> In <40>, -X4- is -CO-CH₂-O- or -CO-NH-(CH₂CH₂O)ₘ-NH-CO-CH₂-O- (m representing an integer of 1 or greater).
<42> In <40> or <41>, the carboxymethyl dextran has the following formula (4) (in which one *R*₁ is CH₂COONa, at least one is a group represented by formula (4)-1, the rest are H, and i' is an integer from 100 to 20,000). 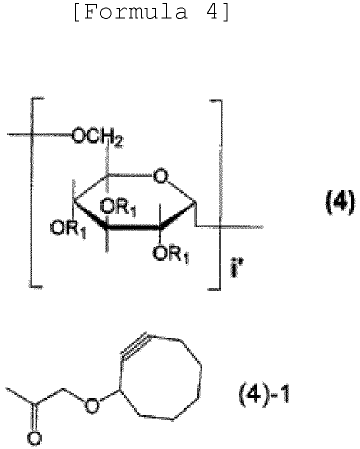
<43> Chitosan whose -NH₂ groups have been partially substituted by a -NH-X3-N₃ group, X3 being an alkylene group comprising at least one substituent selected from the group consisting of an ether group, an ester group, an amide group, a hydrazide group, a disulfide group, and a carbonyl group. X3 is an alkylene group or polysaccharide-derived group comprising an amide group in addition to -NH-. The -NH-X3-N₃ group forms - NH-CO-X31- or -NH-X31- (X31 representing an alkylene group-comprising group or polysaccharide-derived group similar to that of X3), preferably -NH-CO-X31-.
<44> In <43>, -X3- is -CO-(CH₂)₃- or -CO-NH-(CH₂CH₂O)ₘ-NH-CO-(CH₂)₃- (m representing an integer of 1 or greater).
<45> Chitosan whose -NH₂ groups have been substituted by a -NH-X4-(cyclooctyne or cyclooctyne derivative) group, X4 being an alkylene group comprising at least one substituent selected from the group consisting of an ether group, an ester group, an amide group, a hydrazide group, a disulfide group, and a carbonyl group. X4 is an alkylene group or polysaccharide-derived group comprising an amide group in addition to -NH-. The -NH-X4-(cyclooctyne or cyclooctyne derivative) group forms -NH-CO-X41- or -NH-X41- (X41 representing an alkylene group-comprising group or polysaccharide-derived group similar to that of X4).
<46> In <45>, -X4- is -CO-CH₂-O- or -CO-NH-(CH₂CH₂O)ₘ-NH-CO-CH₂-O- (m representing an integer of 1 or greater).
<47> A method of producing hyaluronic acid whose - OH groups have been at least partially substituted by a-O-X3-N₃ group (X3 being a single bond or a group having a molecular weight of 10,000 or less), the method comprising the steps of:
   1) readying hyaluronic acid;
   2) readying a substance containing an azide group and a functional group other than an azide group (non-limiting examples including a carboxyl group, an amino group, a thiol group, a hydrazide group, an aldehyde group, or a hydroxyl group; preferably a carboxyl group, an amino group, or a hydrazide group; more preferably a carboxyl group or an amino group);
   3) ion-substituting the hyaluronic acid from step 1) with a salt comprising a long-chain alkyl ammonium cation (non-limiting examples including a tetrabutylammonium salt, a trioctylmethylammonium salt, or a benzyldimethyloctadecylammonium salt; preferably a tetrabutylammonium salt or trioctylmethylammonium salt; more preferably a tetrabutylammonium salt) that is soluble in both water and an organic solvent, solubilizing the acid in an organic solvent, and preparing a solution of the solubilized acid;
   4) obtaining a solution of the substance from 2) in an organic solvent; and
   5) mixing the solution from step 4) and the solution of solubilized acid from step 3), and reacting the functional group and the OH groups of the hyaluronic acid via a carboxylation reaction (non-limiting examples including carbodiimidization and imidazolation; preferably imidazolation);
      thereby obtaining the hyaluronic acid.
<48> In <47>, the functional group of the substance from step 2) is a carboxylic acid group, and
   the substance is obtained by including steps of:
   2)-1) readying a compound having a molecular weight of 10,000 or less, preferably 5,000 or less, more preferably 3,000 or less, one end of the compound comprising an amino group, and the other end comprising a carboxylic acid group; and
   2)-2) reacting the compound with an azidate (non-limiting examples including imidazole-1-sulfonyl azide and triflic azide; preferably imidazole-1-sulfonyl azide) in a solvent in the presence of a catalyst;
<49> A method of producing hyaluronic acid whose - OH groups have been at least partially substituted by a - O-X4-(cyclooctyne or cyclooctyne derivative) group (X4 representing a single bond or group having a molecular weight of 10,000 or less); the method comprising the steps of:
   1) readying hyaluronic acid;
   2') readying a substance containing a cyclooctyne group and a functional group other than a cyclooctyne group (non-limiting examples including a carboxyl group, an amino group, a thiol group, a hydrazide group, an aldehyde group, or a hydroxyl group; preferably a carboxyl group, an amino group, or a hydrazide group; more preferably a carboxyl group or an amino group);
   3) ion-substituting the hyaluronic acid from step 1) with a salt comprising a long-chain alkyl ammonium cation (non-limiting examples including a tetrabutylammonium salt, a trioctylmethylammonium salt, or a benzyldimethyloctadecylammonium salt; preferably a tetrabutylammonium salt or trioctylmethylammonium salt; more preferably a tetrabutylammonium salt) that is soluble in both water and an organic solvent, solubilizing the acid in an organic solvent, and preparing a solution of the solubilized acid;
   4) obtaining a solution of the substance from 2) in an organic solvent; and
   5) mixing the solution from step 4) and the solution of solubilized acid from step 3), and reacting the functional group and the OH groups of the hyaluronic acid via a carboxylation reaction (non-limiting examples including carbodiimidization and imidazolation; preferably imidazolation);
      thereby obtaining the hyaluronic acid.
<50> In <49>, the functional group of the substance from step 2') is a carboxylic acid group, and the substance is obtained by including steps of:
   2')-1) reacting a substance comprising a hydroxyl group and an ester group (non-limiting examples including methyl glycolate, methyl lactate, or tert-butyl-4-hydroxy butyrate; preferably methyl glycolate or methyl lactate; more preferably methyl glycolate) and a bromoform adduct of cycloheptene in an organic solvent in the presence of a catalyst (a non-limiting example being silver trifluoromethanesulfonate) to obtain a substance comprising a 1-bromocyclooctene group and an ester group;
   2')-2) converting the substance comprising a 1-bromocyclooctene group and an ester group to a debrominated alkyne in a solvent to obtain a substance comprising a cyclooctyne group and an ester group; and 2')-3) subjecting the substance comprising a cyclooctyne group and an ester group to a hydrolyzing reaction.
<51> A method of producing carboxymethyl dextran whose -OH groups have been at least partially substituted by a -O-X3-N₃ group (X3 being a single bond or a group having a molecular weight of 10,000 or less), the method comprising the steps of:
   1') readying carboxymethyl dextran;
   2) readying a substance containing an azide group and a functional group other than an azide group (non-limiting examples including a carboxyl group, an amino group, a thiol group, a hydrazide group, an aldehyde group, or a hydroxyl group; preferably a carboxyl group, an amino group, or a hydrazide group; more preferably a carboxyl group or an amino group);
   3') ion-substituting the carboxymethyl dextran from step 1') with a salt comprising a long-chain alkyl ammonium cation (non-limiting examples including a tetrabutylammonium salt, a trioctylmethylammonium salt, or a benzyldimethyloctadecylammonium salt; preferably a tetrabutylammonium salt or trioctylmethylammonium salt; more preferably a tetrabutylammonium salt) that is soluble in both water and an organic solvent, solubilizing the carboxymethyl dextran in an organic solvent, and preparing a solution of the solubilized carboxymethyl dextran;
   4) obtaining a solution of the substance from 2) in an organic solvent; and
   5') mixing the solution from step 4) and the solution of solubilized carboxymethyl dextran from step 3), and reacting the functional group and the OH groups of the carboxymethyl dextran via a carboxylation reaction (non-limiting examples including carbodiimidization and imidazolation; preferably imidazolation);
      thereby obtaining the carboxymethyl dextran.
<52> In <51>, the functional group of the substance from step 2) is a carboxylic acid group, and
   the substance is obtained by including steps of:
   2)-1) readying a compound having a molecular weight of 10,000 or less, preferably 5,000 or less, more preferably 3,000 or less, one end of the compound comprising an amino group, and the other end comprising a carboxylic acid group; and
   2)-2) reacting the compound with an azidate (non-limiting examples including imidazole-1-sulfonyl azide and triflic azide; preferably imidazole-1-sulfonyl azide) in a solvent in the presence of a catalyst;
<53> A method of producing carboxymethyl dextran whose -OH groups have been at least partially substituted by a -O-X4-(cyclooctyne or cyclooctyne derivative) group (X4 representing a single bond or group having a molecular weight of 10,000 or less); the method comprising the steps of:
   1') readying carboxymethyl dextran;
   2') readying a substance containing a cyclooctyne group and a functional group other than a cyclooctyne group (non-limiting examples including a carboxyl group, an amino group, a thiol group, a hydrazide group, an aldehyde group, or a hydroxyl group; preferably a carboxyl group, an amino group, or a hydrazide group; more preferably a carboxyl group or an amino group);
   3') ion-substituting the carboxymethyl dextran from step 1') with a salt comprising a long-chain alkyl ammonium cation (non-limiting examples including a tetrabutylammonium salt, a trioctylmethylammonium salt, or a benzyldimethyloctadecylammonium salt; preferably a tetrabutylammonium salt or trioctylmethylammonium salt; more preferably a tetrabutylammonium salt) that is soluble in both water and an organic solvent, solubilizing the acid in an organic solvent, and preparing a solution of the solubilized acid;
   4) obtaining a solution of the substance from 2) in an organic solvent; and
   5') mixing the solution from step 4) and the solution of solubilized acid from step 3'), and reacting the functional group and the OH groups of the carboxymethyl dextran via a carboxylation reaction (non-limiting examples including carbodiimidization and imidazolation; preferably imidazolation);
      thereby obtaining the carboxymethyl dextran of ').
<54> In <53>, the functional group of the substance from step 2') is a carboxylic acid group, and
   the substance is obtained by including steps of:
   2')-1) reacting a substance comprising a hydroxyl group and an ester group (non-limiting examples including methyl glycolate, methyl lactate, or tert-butyl-4-hydroxy butyrate; preferably methyl glycolate or methyl lactate; more preferably methyl glycolate) and a bromoform adduct of cycloheptene in an organic solvent in the presence of a catalyst (a non-limiting example being silver trifluoromethanesulfonate) to obtain a substance comprising a 1-bromocyclooctene group and an ester group;
   2')-2) converting the substance comprising a 1-bromocyclooctene group and an ester group to a debrominated alkyne in a solvent to obtain a substance comprising a cyclooctyne group and an ester group; and 2')-3) subjecting the substance comprising a cyclooctyne group and an ester group to a hydrolyzing reaction.

### ADVANTAGES OF THE INVENTION

In accordance with the present invention, it is possible to provide a novel hydrogel, particularly a novel hydrogel using a polysaccharide as a polymer skeleton, and more particularly to a novel *in situ* crosslinking hydrogel exhibiting biocompatibility in in *vivo* environments. Specifically, the present invention yields the effect that an *in situ* crosslinking hydrogel can be efficiently formed *in vivo* by injecting two types of polymer component into the body via a simple operation.

In accordance with the present invention, it is also possible, apart from or in addition to the abovementioned effect, to provide a material comprising the hydrogel, especially a biocompatible material. With respect to this point, the biocompatible material according to the present invention is advantageous over a conventional hydrogel of polyethylene glycol or the like in that a biocompatible material exhibiting superior biodegradability can be provided.

Furthermore, in accordance with the present invention, it is also possible, apart from or in addition to the abovementioned effects, to provide a method of producing the hydrogel.

In accordance with the present invention, it is also possible, apart from or in addition to the abovementioned effects, to provide a drug constituting a feedstock for the hydrogel, as well as a method of producing the same.

### BRIEF DESCRIPTION OF THE DRAWINGS

[FIG. 1] FIG. 1 is a schematic illustration of the structure of a hydrogel according to the present application.
[FIG. 2] FIG. 2 is a schematic illustration of a double syringe.
[FIG. 3] FIG. 3 is an illustration of a tissue stain in which the hydrogel according to the present application has been hypodermically injected.

### BEST MODE FOR CARRYING OUT THE INVENTION

The invention according to the present application will be described in detail hereafter.

The present application provides a hydrogel, a method of producing the hydrogel, a specific hyaluronic acid or specific carboxymethyl dextran constituting a feedstock for the hydrogel, and a method of producing the feedstock. These will be described in order hereafter.

### <Hydrogel>

The present application provides a hydrogel comprising:
a) a first polymer section selected from the group consisting of hyaluronic acid, carboxymethyl dextran, cellulose derivatives (e.g., carboxymethyl cellulose), and chitosan;
b) a second polymer section, selected from the group consisting of hyaluronic acid, carboxymethyl dextran, cellulose derivatives (e.g., carboxymethyl cellulose), and chitosan, that may be the same or different from a), but having a different molecule; and
c) a triazole ring group or derivative group thereof;
   the hydrogel having a structure such that the a) first polymer section and b) second polymer section are crosslinked via the mediation of the c) triazole ring group or derivative group thereof.

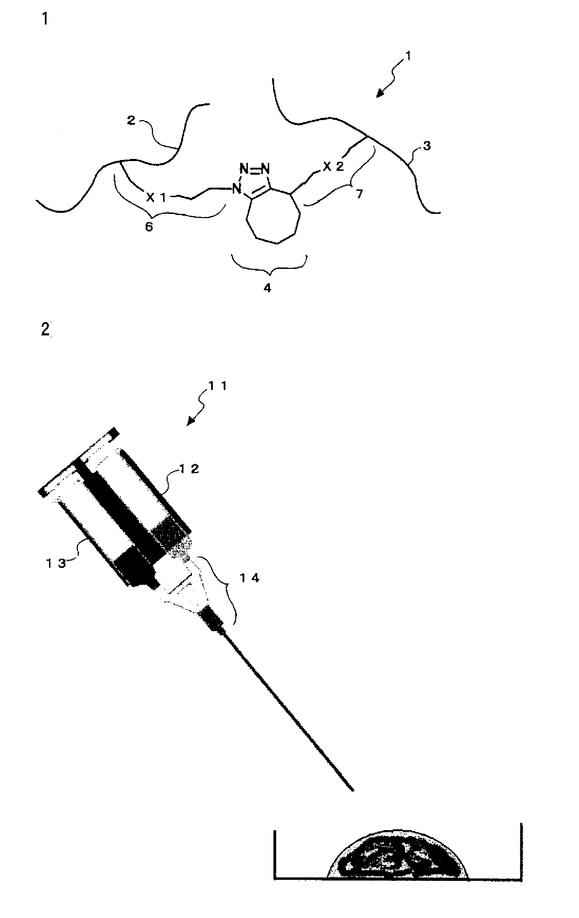

The hydrogel has the schematic structure 1 shown in FIG. 1, centering upon the c) triazole ring group or derivative group thereof as the point of crosslinkage.

In other words, FIG. 1 is a schematic illustration of the structure 1 of the hydrogel according to the present application.

The structure 1 of the hydrogel comprises a first polymer section 2, a second polymer section 3, and a triazole ring group or derivative group thereof 4. In FIG. 1, only a triazole ring group will be shown for 4 for the sake of simplicity.

The first polymer section 2 and the second polymer section 3 are crosslinked by the triazole ring group 4. A spacer group X1 is present between the first polymer section 2 and the triazole ring group 4. A spacer group X2 is present between the second polymer section and the triazole ring group 4. X1 and X2 will be described hereafter.

The first polymer section is selected from the group consisting of hyaluronic acid, carboxymethyl dextran, cellulose derivatives (e.g., carboxymethyl cellulose), and chitosan; hyaluronic acid, carboxymethyl dextran, or chitosan being preferable.

The second polymer section may be of the same or a different type as the first polymer section, but has a different molecule from the first polymer section. The second polymer section is selected from the group consisting of hyaluronic acid, carboxymethyl dextran, cellulose derivatives (e.g., carboxymethyl cellulose), and chitosan; hyaluronic acid, carboxymethyl dextran, or chitosan being preferable.

The triazole ring group is as shown by 4 in FIG. 1; non-limiting examples of derivative groups thereof include monofluorinated cyclooctyl groups, difluorinated octyl groups, and other groups in which the cyclooctyl group of the triazole is substituted by a halogen group; or dimethoxy azocyclooctyl groups and other groups disclosed in PROCEEDINGS OF THE NATIONAL ACADEMY OF SCIENCES OF THE UNITED STATES OF AMERICA, vol. 107, 5, pp. 1821-1826 (2010) (the entirety of which is incorporated herein for reference).

The first and second polymer sections are selected from the above, and comprise an OH group and/or a -NH₂ group. The triazole ring group or derivative group thereof is present in an amount equivalent to 5-60 mol%, preferably 10-50 mol%, more preferably 20-30 mol%, taking the total amount of -OH groups or total amount of -NH₂ groups in the first polymer section as 100 mol%. The triazole ring group or derivative group thereof is also present in an amount equivalent to 5-60 mol%, preferably 10-50 mol%, more preferably 20-30 mol%, taking the total amount of -OH groups or total amount of -NH₂ groups in the second polymer section as 100 mol%.

A spacer group X1 may be present between the first polymer section and the triazole ring group or derivative group thereof. X1 represents a single bond or a group having a molecular weight of 10,000 or less. X1 is preferably an alkylene group or polysaccharide-derived group comprising at least one substituent selected from the group consisting of an ether group, an ester group, an amide group, a hydrazide group, a disulfide group, and a carbonyl group, more preferably an alkylene group or polysaccharide-derived group comprising an ester group, an ether group, or an amide group.

The first polymer section is selected from the group described above, and comprises a -OH group and/or a -NH₂ group. X1 is substituted for the -OH group to form a bond, thereby forming -O-X1-, preferably -O-CO-X11-, -CO-NH-X11-, -CO-NH-NH-X11-, -S-S-X11-, or -CO-X11- (X11 representing an alkylene group or polysaccharide-derived group similar to that of X1 described above), more preferably -O-CO-X11- or -CO-NH-X11-.

The -OH group is substituted by a -COOH group, a -SH group, or another suitable group, which may be further substituted to form the abovementioned group.

If the first polymer section comprises a -COOY group (Y signifying a monovalent cation of hydrogen, sodium, or the like), the group is substituted to form a bond, thereby forming -CO-X1-, preferably -CO-NH-X11-, - CO-NH-NH-X11-, or -CO-O-X11- (X11 representing an alkylene group or polysaccharide-derived group similar to that of X1 above), more preferably -CO-O-X11- or -CO-NH-X11-. If the first polymer section comprises a -NH₂ group, the group is substituted to form a bond, thereby forming -NH-X1-, preferably -NH-CO-X11- or -NH-CO-NH-X11-(X11 being defined similarly to X1 above).

A spacer group X2 may be present between the second polymer section and the triazole ring group or derivative group thereof. X2 represents a single bond or a group having a molecular weight of 10,000 or less. X2 is preferably an alkylene group or polysaccharide-derived group comprising at least one substituent selected from the group consisting of an ether group, an ester group, an amide group, a hydrazide group, a disulfide group, and a carbonyl group, more preferably an alkylene group or polysaccharide-derived group comprising an ester group, an ether group, or an amide group.

The second polymer section is selected from the group described above, and comprises a -OH group and/or a -NH₂ group. X2 is substituted for the OH group to form a bond, forming -O-X2-, preferably -O-CO-X21-, -CO-NH-X21-, -CO-NH-NH-X21-, -S-S-X21-, or -CO-X21- (X21 representing a alkylene group or polysaccharide-derived group similar to that of X2 above), more preferably -O-CO-X21- or -CO-NH-X21-.

The -OH group is substituted by a -COOH group, a -SH group, or another suitable group, which may be further substituted to form the abovementioned group.

If the second polymer section comprises a -COOY group (Y signifying a monovalent cation of hydrogen, sodium, or the like), the group is substituted to form a bond, thereby forming -CO-X2-, preferably -CO-NH-X21-, - CO-NH-NH-X21-, or -CO-O-X21- (X21 representing an alkylene group or polysaccharide-derived group similar to that of X2 above), more preferably -CO-O-X21- or -CO-NH-X21-. If the second polymer section comprises a -NH₂ group, the group is substituted to form a bond, thereby forming -NH-X2-, preferably -NH-CO-X21- or -NH-CO-NH-X21-(X21 being defined similarly to X2 above).

Because of its high level of biocompatibility, the hydrogel according to the present application can be used for medical materials, cosmetic surgical materials, food materials, cosmetic materials, and the like. In particular, the hydrogel according to the present application can be used in a biocompatible material comprising the hydrogel. More specific, non-limiting examples of biocompatible materials include adhesion barriers, drug delivery system carriers, cell-encapsulating materials, cell culture dish coating agents, and the like.

An adhesion barrier can be used as described hereafter to prevent adhesions from forming within the body.

Specifically, a hydrogel can be formed via steps of:
A) readying a) a first liquid containing hyaluronic acid comprising an azide group, carboxymethyl dextran comprising an azide group, or chitosan comprising an azide group;
B) readying a b) second liquid containing hyaluronic acid comprising a cyclooctyne group or a cyclooctyne group derivative, carboxymethyl dextran comprising a cyclooctyne group or a cyclooctyne group derivative, or chitosan comprising a cyclooctyne group or a cyclooctyne group derivative; and
C') injecting the a) first liquid and the b) second liquid into an area within the body where adhesions are to be prevented, and mixing the a) first liquid and the b) second liquid;
   thereby forming a hydrogel and allowing adhesions to be prevented via the hydrogel.

A cell- encapsulating material can be used as follows to enclose cells within the body.

Specifically, via steps of:
A) readying a) a first liquid containing hyaluronic acid comprising an azide group, carboxymethyl dextran comprising an azide group, or chitosan comprising an azide group;
B) readying a b) second liquid containing hyaluronic acid comprising a cyclooctyne group or a cyclooctyne group derivative, carboxymethyl dextran comprising a cyclooctyne group or a cyclooctyne group derivative, or chitosan comprising a cyclooctyne group or a cyclooctyne group derivative; and
C") injecting the a) first liquid and the b) second liquid near target cells within the body, and mixing the a) first liquid and the b) second liquid;
   a hydrogel can be formed, and the hydrogel used to enclose target cells.

### <Method of producing hydrogel>

The hydrogel can be produced via a method such as the following.

Specifically, via steps of: A) readying a liquid containing a) a first polymer selected from the group consisting of hyaluronic acid, carboxymethyl dextran, cellulose derivatives (e.g., carboxymethyl cellulose), and chitosan, the first polymer comprising an azide group;
B) readying a liquid comprising b) a second polymer, selected from the group consisting of hyaluronic acid, carboxymethyl dextran, cellulose derivatives (e.g., carboxymethyl cellulose), and chitosan, that may be the same as or different from a), but having a different molecule than a), the second polymer comprising a cyclooctyne group or a cyclooctyne group derivative; and
C) mixing a) the first liquid and b) the second liquid;
   thereby inducing a click reaction between the azide group and the cyclooctyne group or cyclooctyne group derivative, thus forming a triazole ring or derivative thereof, a hydrogel having a structure in which the first polymer and the second polymer are crosslinked via the mediation of the triazole ring or derivative thereof can be formed to obtain a hydrogel. Step C) of this process can be performed *in vivo,* as described above.

Steps A) and B) are steps of readying liquids containing the first and second polymers, respectively.

The first polymer is selected from the group consisting of hyaluronic acid, carboxymethyl dextran, cellulose derivatives (e.g., carboxymethyl cellulose), and chitosan, and comprises an azide group.

The second polymer is also selected from the group consisting of hyaluronic acid, carboxymethyl dextran, cellulose derivatives (e.g., carboxymethyl cellulose), and chitosan, and comprises an azide group.

The preparation of the first and second polymers will be described hereafter.

The liquid containing the first polymer can be obtained by dissolving the obtained first polymer in phosphate buffered saline (PBS), a tris buffer solution, a citrate buffer solution, a carbonate-bicarbonate buffer solution, Hanks' solution, physiological saline, Dulbecco's modified Eagle's medium (DMEM), Roswell Park Memorial Institute 1640 (RPMI 1640) culture medium, or another solvent.

Like the liquid containing the first polymer, the liquid containing the second polymer can be obtained by dissolving the obtained first polymer in phosphate buffered saline (PBS), a tris buffer solution, a citrate buffer solution, a carbonate-bicarbonate buffer solution, Hanks' solution, physiological saline, Dulbecco's modified Eagle's medium (DMEM), Roswell Park Memorial Institute (RPMI 1640) culture medium, or another solvent (the solvent used in the liquid containing the second polymer may be identical to or different from the solvent used in the liquid containing the first polymer).

The respective concentrations of the liquids containing the first and second polymers depend upon the first and second polymers used, the solvent, the desired gelling type, and the like, but will be 0.01-20%, preferably 0.5-10%, more preferably 1-5%.

Step C) is a step of mixing the liquids obtained in steps A) and B).

The mixing can be performed via various methods. If the mixture is disposed at a desired position and a gel formed at that position, a double syringe is preferably used to perform the mixing step C).

FIG. 2 is a schematic illustration of a double syringe 11.

The double syringe 11 comprises a syringe 12 and a syringe 13 adjacently disposed, and a mixing/discharging part 14, coupling the discharging parts of the syringes, for mixing and discharging the liquid within the syringe 12 and the liquid within the syringe 13. FIG. 2 merely shows one example of the double syringe; other configurations are possible so long as they are capable of mixing the two liquids and discharging the mixed liquid at a desired position.

If a double syringe is used, one half thereof is filled with the liquid comprising the a) first polymer and the other half with the liquid containing the b) second polymer, and the liquid containing the a) first polymer and the liquid containing the b) second polymer are dispensed from the double syringe roughly simultaneously.

C) The mixing process used will depend upon the first and second polymers used, the solvent, and so forth, but is performed at a temperature in a range from the human body temperature of 37°C to a room temperature of 25°C and ambient pressure.

In a preferred aspect, the steps A) - C) can be performed using a kit. For example, the kit contains at least suitable amounts of the liquid containing the first polymer and the liquid containing the second polymer. As such, such a kit is encompassed by the scope of the present invention.

There is a spacer group X1 (X1 representing a single bond or a group having a molecular weight of 10,000 or less) between the first polymer and the azide group.

X1 is preferably an alkylene group or polysaccharide-derived group comprising at least one substituent selected from the group consisting of an ether group, an ester group, an amide group, a hydrazide group, a disulfide group, and a carbonyl group, more preferably an alkylene group or polysaccharide-derived group comprising an ester group, an ether group, or an amide group.

The first polymer is selected from the group described above, and comprises a -OH group and/or a -NH₂ group. X1 is substituted for the -OH group to form a bond, thereby forming -O-X1-, preferably -O-CO-X11-, -CO-NH-X11-, -CO-NH-NH-X11-, -S-S-X11-, or -CO-X11- (X11 representing an alkylene group or polysaccharide-derived group similar to that of X1 described above), more preferably -O-CO-X11- or -CO-NH-X11-. In particular,-X1- is preferably -CO-(CH₂)₃- or -CO-NH-(CH₂CH₂O)ₘ-NH-CO-CH₂-O-.

The -OH group is substituted by a -COOH group, a -SH group, or another suitable group, which may be further substituted to form the abovementioned group.

If the first polymer comprises a -COOY group (Y signifying a monovalent cation of hydrogen, sodium, or the like), the group may be substituted to form a bond, thereby forming -CO-X1-, preferably -CO-NH-X11-, -CO-NH-NH-X11-, or -CO-O-X11- (X11 representing an alkylene group or polysaccharide-derived group similar to that of X1 above), more preferably -CO-O-X11- or -CO-NH-X11-. If the first polymer section comprises a -NH₂ group, the group is substituted to form a bond, thereby forming -NH-X1-, preferably -NH-CO-X11- or -NH-CO-NH-X11- (X11 being defined similarly to X1 above).

The azide group is present in an amount equivalent to 5-60 mol%, preferably 10-50 mol%, more preferably 20-30 mol%, taking the total amount of -OH groups or total amount of -NH₂ groups in the first polymer as 100 mol%.

There is a spacer group X2 (X2 representing a single bond or a group having a molecular weight of 10,000 or less) between the second polymer and the cyclooctyne group or cyclooctyne group derivative.

X2 is preferably an alkylene group or polysaccharide-derived group comprising at least one substituent selected from the group consisting of an ether group, an ester group, an amide group, a hydrazide group, a disulfide group, and a carbonyl group, more preferably an alkylene group-comprising group or polysaccharide-derived group comprising an ester group, an ether group, or an amide group.

The second polymer is selected from the group described above, and comprises a -OH group and/or a NH₂ group. X2 is substituted for the OH group to form a bond, forming -O-X2-, preferably -O-CO-X21-, -CO-NH-X21-, -CO-NH-NH-X21-, -S-S-X21-, or -CO-X21- (X21 representing a alkylene group or polysaccharide-derived group similar to that of X2 above), more preferably -O-CO-X21- or -CO-NH-X21-. In particular, -X2- is preferably -CO-CH₂-O- or - CO-NH-(CH₂CH₂O)ₘ-NH-CO-CH₂-O-.

The -OH group is substituted by a -COOH group, a -SH group, or another suitable group, which may be further substituted to form the abovementioned group.

If the second polymer comprises a -COOY group (Y signifying a monovalent cation of hydrogen, sodium, or the like), the group may be substituted to form a bond, thereby forming -CO-X2-, preferably -CO-NH-X21-, -CO-NH-NH-X21-, or -CO-O-X21- (X21 representing an alkylene group or polysaccharide-derived group similar to that of X1 above), more preferably -CO-O-X21- or -CO-NH-X21-. If the second polymer section comprises a -NH₂ group, the group is substituted to form a bond, thereby forming -NH-X2-, preferably -NH-CO-X21- or -NH-CO-NH-X21- (X21 being defined similarly to X1 above).

The cyclooctyne group or cyclooctyne group derivative is present in an amount equivalent to 5-60 mol%, preferably 10-50 mol%, more preferably 20-30 mol%, taking the total amount of -OH groups or total amount of -NH₂ groups in the b) second polymer as 100 mol%.

The cyclooctyne group derivative has the same definition as given above.

<Specific hyaluronic acid constituting hydrogel feedstock>

The present application provides a specific hyaluronic acid constituting a feedstock for the hydrogel.

### «Hyaluronic acid comprising azide group»

The present application provides hyaluronic acid comprising an azide group; specifically, hyaluronic acid whose -OH groups are at least partially substituted by an -O-X3-N₃ group (X3 being a single bond or a group having a molecular weight of 10,000 or less).

X3 is an alkylene group or polysaccharide-derived group comprising an ether group or an ester group in addition to -O-. The -O-X3-N₃ group forms -O-CO-X31- or -O-X31- (X31 representing an alkylene group or polysaccharide-derived group), preferably -O-CO-X31-. In particular, -X3- is -CO-(CH₂)₃- or -CO-NH-(CH₂CH₂O)ₘ-NH-CO-(CH₂)₃- (m representing an integer of 1 or greater).

The hyaluronic acid comprising the azide group has the following formula (1). In formula (1), at least one R is a group represented by the formula (1)-1, the rest are H, and n represents an integer from 100 to 20,000.

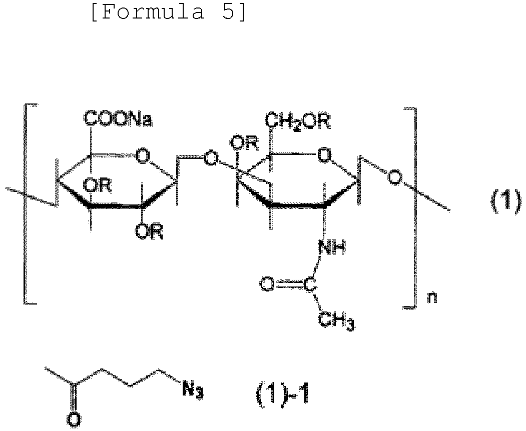

### «Hyaluronic acid comprising cyclooctyne group or cyclooctyne derivative group»

The present application provides hyaluronic acid comprising a cyclooctyne group or cyclooctyne derivative group; specifically, hyaluronic acid whose -OH groups are at least partially substituted by a -O-X4-(cyclooctyne or cyclooctyne derivative) group (X4 representing a single bond or a group having a molecular weight of 10,000 or less).

Non-limiting examples of cyclooctyne derivative groups include monofluorinated cyclooctyne groups, difluorinated cyclooctyne groups, and other groups in which the cyclooctyne groups are partially substituted by a halogen group; and dimethoxy azocyclooctyne groups and other groups disclosed in PROCEEDINGS OF THE NATIONAL ACADEMY OF SCIENCES OF THE UNITED STATES OF AMERICA, vol. 107, 5, pp. 1821-1826 (2010) (the entirety of which is incorporated herein for reference).

X4 is an alkylene group or polysaccharide-derived group comprising an ether group or an ester group in addition to -O-. The -O-X4-(cyclooctyne or cyclooctyne derivative) group preferably forms -O-CO-X41- or -O-X41-(X41 being an alkylene group or polysaccharide-derived group), more preferably -O-CO-X41-. In particular, -X4-is -CO-CH₂-O- or -CO-NH-(CH₂CH₂O)ₘ-NH-CO-CH₂-O- (m representing an integer of 1 or greater).

The hyaluronic acid comprising the cyclooctyne group or cyclooctyne derivative group has the following formula (2). In formula (2), at least one R is a group represented by the formula (2)-1), the rest are H, and n' represents an integer from 100 to 20,000.

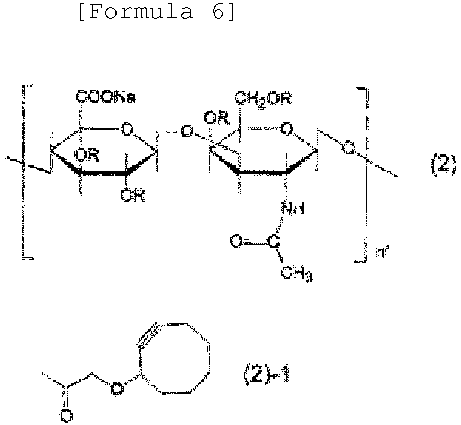

### <Specific carboxymethyl dextran constituting hydrogel feedstock>

The present application provides a specific carboxymethyl dextran constituting a feedstock for the hydrogel.

### «Carboxymethyl dextran comprising azide group»

The present application provides carboxymethyl dextran comprising an azide group; specifically, carboxymethyl dextran whose -OH groups are at least partially substituted by an -O-X3-N₃ group (X3 being a single bond or a group having a molecular weight of 10,000 or less).

X3 is an alkylene group or polysaccharide-derived group comprising an ether group or an ester group in addition to -O-. The -O-X3-N₃ group forms -O-CO-X31- or -O-X31- (X31 representing an alkylene group or polysaccharide-derived group), preferably -O-CO-X31-. In particular, -X3- is -CO-(CH₂)₃- or -CO-NH-(CH₂CH₂O)ₘ-NH-CO-(CH₂)₃- (m representing an integer of 1 or greater).

The carboxymethyl dextran comprising the azide group has the following formula (3). In formula (3), one *R*₁ is CH₂COONa, at least one is a group represented by the formula (3)-1, the rest are H, and i represents an integer from 100 to 20,000.

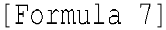

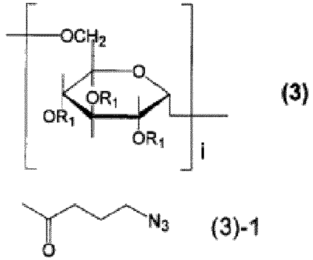

### «Carboxymethyl dextran comprising cyclooctyne group or cyclooctyne derivative group»

The present application provides carboxymethyl dextran comprising a cyclooctyne group or cyclooctyne derivative group; specifically, carboxymethyl dextran whose -OH groups are at least partially substituted by a -O-X4-(cyclooctyne or cyclooctyne derivative) group (X4 representing a single bond or a group having a molecular weight of 10,000 or less).

X4 is an alkylene group or polysaccharide-derived group comprising an ether group or an ester group in addition to -O-. The -O-X4-(cyclooctyne or cyclooctyne derivative) group preferably forms -O-CO-X41- or -O-X41-(X41 being an alkylene group-comprising group or polysaccharide-derived group), more preferably -O-CO-X41-. In particular, -X4- is -CO-CH₂-O- or -CO-NH-(CH₂CH₂O)ₘ-NH-CO-CH₂-O- (m representing an integer of 1 or greater).

The carboxymethyl dextran comprising the cyclooctyne group or cyclooctyne derivative group has the following formula (4). In formula (4), one *R*₁ is CH₂COONa, at least one is a group represented by the formula (4)-1, the rest are H, and i' represents an integer from 100 to 20,000.

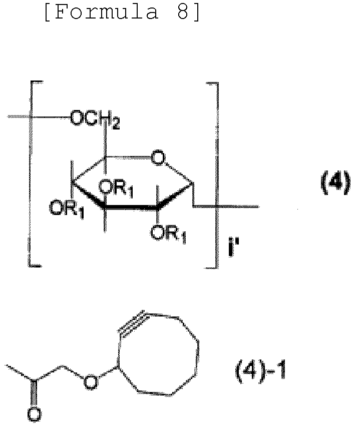

### <Specific chitosan constituting hydrogel feedstock>

The present application provides a specific chitosan constituting a feedstock for the hydrogel.

### «Chitosan comprising azide group»

The present application provides chitosan comprising an azide group; specifically, chitosan whose -NH₂ groups have been at least partially substituted by a -NH-X3-N₃ group, X3 being an alkylene group comprising at least one substituent selected from the group consisting of an ether group, an ester group, an amide group, a hydrazide group, a disulfide group, and a carbonyl group. X3 is an alkylene group or polysaccharide-derived group comprising an amide group in addition to -NH-. The -NH-X3-N₃ group forms -NH-CO-X31- or -NH-X31- (X31 representing an alkylene group-comprising group or polysaccharide-derived group similar to that of X3), preferably -NH-CO-X31-. In particular, -X3- is -CO-(CH₂)₃- or -CO-NH-(CH₂CH₂O)ₘ-NH-CO-(CH₂)₃- (m representing an integer of 1 or greater).

### «Chitosan comprising cyclooctyne group or cyclooctyne derivative group»

The present application provides carboxymethyl dextran comprising a cyclooctyne group or cyclooctyne derivative group; specifically, chitosan whose -NH₂ groups have been substituted by a -NH-X4-(cyclooctyne or cyclooctyne derivative) group, X4 being an alkylene group comprising at least one substituent selected from the group consisting of an ether group, an ester group, an amide group, a hydrazide group, a disulfide group, and a carbonyl group. X4 is an alkylene group or polysaccharide-derived group comprising an amide group in addition to -NH-. The -NH-X4-(cyclooctyne or cyclooctyne derivative) group forms -NH-CO-X41- or -NH-X41- (X41 representing an alkylene group-comprising group or polysaccharide-derived group similar to that of X4). In particular, -X4- is -CO-CH₂-O- or -CO-NH-(CH₂CH₂O)ₘ-NH-CO-CH₂-O- (m representing an integer of 1 or greater).

### <Method of producing specific hyaluronic acid constituting hydrogel feedstock<

The present application provides a method of producing a specific hyaluronic acid constituting a feedstock for the hydrogel.

### «Method of producing hyaluronic acid comprising an azide group»

The hyaluronic acid comprising an azide group described above can be produced according to the following method.

Specifically, via steps of:
1) readying hyaluronic acid;
2) readying a substance containing an azide group and a functional group other than an azide group (non-limiting examples including a carboxyl group, an amino group, a thiol group, a hydrazide group, an aldehyde group, or a hydroxyl group; preferably a carboxyl group, an amino group, or a hydrazide group; more preferably a carboxyl group or an amino group);
3) ion-substituting the hyaluronic acid from step 1) with a salt comprising a long-chain alkyl ammonium cation (non-limiting examples including a tetrabutylammonium salt, a trioctylmethylammonium salt, or a benzyldimethyloctadecylammonium salt; preferably a tetrabutylammonium salt or trioctylmethylammonium salt; more preferably a tetrabutylammonium salt) that is soluble in both water and an organic solvent, solubilizing the acid in an organic solvent, and preparing a solution of the solubilized acid;
4) obtaining a solution of the substance from 2) in an organic solvent; and
5) mixing the solution from step 4) and the solution of solubilized acid from step 3), and reacting the functional group and the OH groups of the hyaluronic acid via a carboxylation reaction (non-limiting examples including carbodiimidization and imidazolation; preferably imidazolation);

it is possible to obtain hyaluronic acid comprising an azide group.

Step 1) is a step of readying hyaluronic acid. The hyaluronic acid may be readied by being prepared or by being commercially purchased.

Step 2) is a step of readying a substance comprising an azide group and a functional group other than an azide group apart from step 1). The substance is hyaluronic acid comprising an azide group, in which the substance is a location constituting a spacer group X3 and an azide group. In particular, if the functional group of the substance is a carboxylic acid group, the substance can be prepared as follows. Specifically, the substance can be obtained via steps of:
2)-1) readying a compound having a molecular weight of 10,000 or less, preferably 5,000 or less, more preferably 3,000 or less, one end of the compound comprising an amino group, and the other end comprising a carboxylic acid group; and
2)-2) reacting the compound with an azidate (non-limiting examples including imidazole-1-sulfonyl azide and triflic azide; preferably imidazole-1-sulfonyl azide) in a solvent in the presence of a catalyst;

Non-limiting examples of the compound of step 2)-1) include glycine, gamma-aminobutyric acid, 4-amino-4-oxobutanoic acid, gabapentin, and aminothioacetic acid. The compound of step 2)-1) is preferably glycine or gamma-aminobutyric acid, more preferably gamma-aminobutyric acid.

Non-limiting examples of the solvent of step 2)-2) include methanol, ethanol, isopropyl alcohol, chloroform, and methylene chloride. The solvent of step 2)-2) is preferably methanol or ethanol, more preferably methanol.

A non-limiting example of a catalyst is copper sulfate.

Non-limiting examples of azide compounds include imidazole-1-sulfonyl azide and triflic azide. Imidazole-1-sulfonyl azide is preferable.

Step 2)-2) is performed at a temperature of 0°C-4°C, depending upon the compound used, the solvent used, the catalyst used, and the azide compound used.

Step 3) is a step of ion-substituting the hyaluronic acid using a salt comprising a long-chain alkyl ammonium cation that is soluble in both water and an organic solvent, solubilizing the acid in an organic solvent, and preparing a solution of the solubilized acid.

Non-limiting examples of the salt comprising a long-chain alkyl ammonium cation that is soluble in both water and an organic solvent include a tetrabutylammonium salt, a trioctylmethylammonium salt, or a benzyldimethyloctadecylammonium salt; preferably a tetrabutylammonium salt or trioctylmethylammonium salt; more preferably a tetrabutylammonium salt.

Non-limiting examples of organic solvents used to solubilize the hyaluronic acid include methanol, ethanol, isopropyl alcohol, diethyl ether, dimethyl sulfoxide, diethyl acetamide, tetrahydrofuran, dimethyl formamide, dioxane, N-methylpyrrolidone, toluene, and cyclohexane. Ethanol, acetone, tetrahydrofuran, or dimethyl formamide is preferable, and ethanol, acetone, or tetrahydrofuran is more preferable.

Step 3 is performed in at a temperature of 20-40°C and a pH of 4-12 depending upon the salt comprising a long-chain alkyl ammonium cation that is soluble in both water and an organic solvent used, the organic solvent used, and the like.

Step 4) is a step of obtaining a solution of the substance obtained in step 2) in an organic solvent. Non-limiting examples of organic solvents include diethyl ether, dimethyl sulfoxide, diethyl acetamide, tetrahydrofuran, dimethyl formamide, dioxane, N-methylpyrrolidone, toluene, and cyclohexane. Diethyl ether, dimethyl sulfoxide, dimethyl acetamide, tetrahydrofuran, dimethyl formamide, or dioxane is preferable, and dimethyl sulfoxide is more preferable. The organic solvent may be identical to or different from that used in step 3), but dimethyl sulfoxide is preferable in terms of risk posed by residence in the body.

Step 5) is a step of mixing the solution obtained in step 4) and the solution of solubilized hyaluronic acid obtained in step 3) and reacting the functional groups and the hyaluronic acid OH groups via a carboxylation reaction.

Non-limiting examples of carboxylation reactions include a carbodiimidization reaction and an imidazolation reaction. An imidazolation reaction is preferable.

Step 5) is performed at a temperature of 20-40°C and a pH of 4-12, depending upon the solution obtained in step 4) and the solution of solubilized hyaluronic acid obtained in step 3).

«Method of preparing hyaluronic acid comprising cyclooctyne group or cyclooctyne derivative group»

The hyaluronic acid comprising a cyclooctyne group or cyclooctyne derivative group can be produced according to the following method.

Specifically, via steps of:
1) readying hyaluronic acid;
2') readying a substance containing a cyclooctyne group and a functional group other than a cyclooctyne group (non-limiting examples including a carboxyl group, an amino group, a thiol group, a hydrazide group, an aldehyde group, or a hydroxyl group; preferably a carboxyl group, an amino group, or a hydrazide group; more preferably a carboxyl group or an amino group);
3) ion-substituting the hyaluronic acid from step 1) with a salt comprising a long-chain alkyl ammonium cation (non-limiting examples including a tetrabutylammonium salt, a trioctylmethylammonium salt, or a benzyldimethyloctadecylammonium salt; preferably a tetrabutylammonium salt or trioctylmethylammonium salt; more preferably a tetrabutylammonium salt) that is soluble in both water and an organic solvent, solubilizing the acid in an organic solvent, and readying a solution of the solubilized acid;
4) obtaining a solution of the substance from 2) in an organic solvent; and
5) mixing the solution from step 4) and the solution of solubilized acid from step 3), and reacting the functional group and the OH groups of the hyaluronic acid via a carboxylation reaction;

thereby obtaining hyaluronic acid comprising a cyclooctyne group or a cyclooctyne derivative group.

The same holds for steps 1) and 3) - 5).

Step 2') is a step of readying a substance comprising a cyclooctyne group and a functional group other than a cyclooctyne group apart from step 1). The substance is hyaluronic acid comprising a cyclooctyne group and a cyclooctyne group, in which the substance is a location constituting the spacer group X4 and cyclooctyne group or a cyclooctyne group derivative described above.

In particular, if the functional group of the substance of step 2') is a carboxylic acid group, the substance can be prepared as follows.

Specifically, the substance can be obtained via steps of:
2')-1) reacting a substance comprising a hydroxyl group and an ester group (non-limiting examples including methyl glycolate, methyl lactate, or tert-butyl-4-hydroxy butyrate; preferably methyl glycolate or methyl lactate; more preferably methyl glycolate) and a bromoform adduct of cycloheptene in an organic solvent in the presence of a catalyst (a non-limiting example being silver trifluoromethanesulfonate) to obtain a substance comprising a 1-bromocyclooctene group and an ester group;
2')-2) converting the substance comprising a 1-bromocyclooctene group and an ester group to a debrominated alkyne in a solvent to obtain a substance comprising a cyclooctyne group and an ester group; and
2')-3) subjecting the substance comprising a cyclooctyne group and an ester group to a hydrolyzing reaction.

Step 2')-1) is a step of reaction a substance comprising a hydroxyl group and an ester group and a bromoform adduct of cycloheptene in an organic solvent in the presence of a catalyst, and obtaining a substance comprising a 1-bromocyclooctene group and an ester group.

Non-limiting examples of the substance comprising a hydroxyl group and an ester group include methyl glycolate, methyl lactate, and tert-butyl-4-hydroxybutyrate. Methyl glycolate or methyl lactate is preferable, and methyl glycolate is more preferable.

The organic solvent will depend upon the substance comprising a hydroxyl group and an ester group used and the catalyst used; non-limiting examples include toluene, benzene, cyclohexane, and n-hexane.

The catalyst will depend upon the substance comprising a hydroxyl group and an ester group used and the organic solvent used; a non-limiting example is silver trifluoromethanesulfonate.

Step 2')-1) is performed at a temperature of 20-40°C and a pH of 4-12, depending upon the substance comprising a hydroxyl group and an ester group used, the organic solvent used, and the catalyst used.

Step 2')-2) is a step of converting the substance comprising a 1-bromocyclooctene group and an ester group obtained in step 2')-1) to a debrominated alkyne in a solvent to obtain a substance comprising a cyclooctyne group and an ester group.

Non-limiting examples of solvents include toluene, benzene, cyclohexane, and n-hexane.

Step 2')-2) is performed at a temperature of 20-40°C and a pH of 4-12.

Step 2')-3) is a step of subjecting the substance comprising a cyclooctyne group and an ester group obtained in step 2')-2) to a hydrolyzing reaction.

Step 2')-3) is performed at a temperature of 20-40°C and a pH of 4-12.

### <Method of producing specific carboxymethyl dextran constituting hydrogel feedstock>

The present application provides a method of producing a specific carboxymethyl dextran constituting a feedstock for the hydrogel.

A method of producing carboxymethyl dextran comprising an azide group and carboxymethyl dextran comprising a cyclooctyne group or a cyclooctyne derivative group can be provided by using carboxymethyl dextran instead of hyaluronic acid in the method of producing hyaluronic acid comprising an azide group and hyaluronic acid comprising a cyclooctyne group or cyclooctyne derivative group.

### EXAMPLES

The present invention will be described in further detail below on the basis of examples, but the present invention is not limited to these examples.

### Example 1

### <A. Synthesizing hyaluronic acid comprising an azide group>

### «Synthesizing imidazole-1-sulfonyl azide»

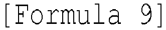

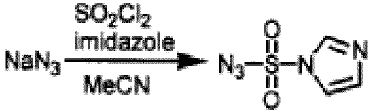

3.20 g (50 mmol) sodium azide was added to a 200 mL eggplant-shaped flask, and 50 mL dehydrated acetonitrile was added thereto and stirred to create a suspension. Next, the suspension was placed in an ice bath and placed in a nitrogen atmosphere. The 4 mL 50 mmol) sulfuryl chloride was added dropwise to the suspension, and the whole was then stirred overnight at room temperature. 6.80 g (0.1 mol) imidazole was added to the suspension in the ice bath, the whole was stirred for 24 hours at room temperature, the reaction solution was transferred to an extraction funnel, and 50 mL ethyl acetate and 100 mL purified water were added to create an extract. The organic phase was left in the funnel, and 100 mL purified water was again added to create an extraction. This extraction process was repeated three times.

100 mL of a saturated sodium hydrogen carbonate aqueous solution was added to the funnel after the aqueous phase had been removed to wash the organic phase. This process was repeated three times.

The organic phase was recovered, magnesium sulfate was added and the whole was dried overnight at room temperature, after which the magnesium sulfate was removed and the solvent was distilled away using an evaporator. Vacuum drying was further performed for several days to obtain a colorless, transparent target product (yield: 5.14 g (29.7 mmol); yield rate: 60.3%).

### «Synthesizing gamma-butyric acid azide»

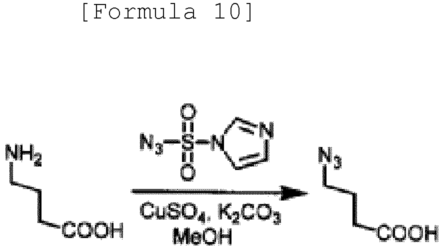

2.41 g (23.4 mmol) gamma-aminobutyric acid, 49.6 mg (0.311 mmol) copper sulfate, and 6.00 g (43.4 mmol) potassium carbonate were added to a 300 mL eggplant-shaped flask. 120 mL methanol was added thereto, and the whole was stirred at room temperature. After stirring for 40 minutes, 4.88 g (28.2 mmol) imidazole-1-sulfonyl azide was added dropwise, and the whole was stirred overnight at room temperature. A few mL of 12 M HCl was added dropwise to lower the pH from approximately 11 to 3. The reaction solution was transferred to a separatory funnel, 250 mL purified water and 200 mL ethyl acetate were added thereto, and the organic phase was extracted. 100 mL ethyl acetate was added to wash the remaining aqueous phase (repeated three times). The organic phase was recovered, magnesium sulfate was added thereto and the whole was dried overnight at room temperature, and the magnesium sulfate was filtered out, after which the solvent was distilled away via evaporation. Vacuum drying was performed to obtain a pale yellow target substance (yield: 3.677 g (28.48 mmol); yield rate: 122%).

### <<Synthesizing azide group-comprising hyaluronic acid A-1>>

Hyaluronic acid anhydrous glucose units (AGU), carbonyl imidazole (CDI), and gamma-butyric acid azide were used at theoretical ratios of 1:2:2.

Hyaluronic acid (Mw = 80,000 Da) was added to an eggplant-shaped flask and dissolved in DMSO.

Gamma-butyric acid azide and CDI were added to a separate flask and dissolved in dimethyl sulfoxide (DMSO) to activate the carboxyl groups of the gamma-butyric acid azide, and a reaction was performed for one hour.

The reaction product was added dropwise to the DMSO solution of hyaluronic acid. The reaction product was heated to 60°C starting 10 minutes prior to being added dropwise, and, after 16-20 hours, heating and stirring was completed, and the whole was left to stand at room temperature. One hour after being left to stand, the whole was dialyzed in 3 L purified water, the water being changed out every hour. After continuing dialysis for three days, the dialysis liquid being replaced every 12 hours, the whole was recovered in a falcon tube and freeze-dried to obtain an azide group-comprising hyaluronic acid A-1. The presence of 10.8 mol% azide groups was confirmed via ¹H-NMR from the ratio of protons derived from the hyaluronic acid (Mw = 80,000 Da) used and protons derived from the azide groups constituting the side chain, taking the total amount of -OH groups in the hyaluronic acid (Mw = 80,000 Da) as 100 mol%.

### Example 2

### <<Synthesizing azide group-comprising hyaluronic acid A-2>>

An azide group-comprising hyaluronic acid A-2 was obtained according to a method similar to that of example 1, except that hyaluronic acid (Mw = 800,000 Da) was used instead of the hyaluronic acid (Mw = 80,000 Da) of example 1. The presence of 12.6 mol% azide groups was confirmed via ¹H-NMR in a manner similar to that of example 1.

### Example 3

### <<Synthesizing azide group-comprising carboxymethyl dextran A-3>>

Azide group-comprising carboxymethyl dextran A-3 was obtained in a manner similar to that of example 1, except that carboxymethyl dextran (Mw = 40,000 Da) was used at an AGU:CDI:gamma-butyric acid azide theoretical ratio of 1:3:3 instead of the hyaluronic acid (Mw = 80,000 Da) of example 1. The presence of 6.9 mol% azide groups was confirmed via ¹H-NMR in a manner similar to that of example 1.

### Example 4

### <<Synthesizing azide group-comprising carboxymethyl dextran A-4>>

Azide group-comprising carboxymethyl dextran A-4 was obtained in a manner similar to that of example 3, except that carboxymethyl dextran (Mw = 160,000 Da) was used instead of the carboxymethyl dextran (Mw = 40,000 Da) of example 3. The presence of 22.2 mol% azide groups was confirmed via ¹H-NMR in a manner similar to that of example 1.

### Example 5

### <<Synthesizing azide group-comprising carboxymethyl dextran A-5>>

Azide group-comprising carboxymethyl dextran A-5 was obtained in a manner similar to that of example 3, except that carboxymethyl dextran (Mw = 580,000 Da) was used instead of the carboxymethyl dextran (Mw = 40,000 Da) of example 3. The presence of 27.5 mol% azide groups was confirmed via ¹H-NMR in a manner similar to that of example 1.

### Example 6

### «Synthesizing azide group-comprising chitosan A-6»

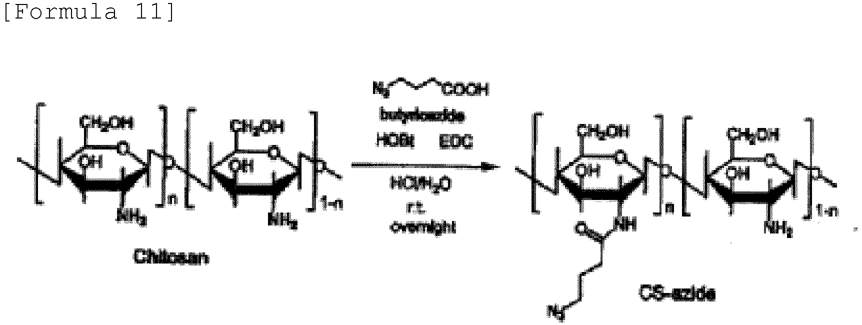

0.2696 g (1.01 mmol per unit) was dissolved in 30 mL 0.1 NHCl in a 100 mL beaker. Complete dissolution was reached after roughly 5 minutes. 0.3848 g (2,98 mmol, 2.9 eq) azide butyric acid was added to a 20 mL eggplant-shaped flask and dissolved in 3 mL DMSO. To this was added 0.4061 g (3.01 mmol, 3.0 eq) HOBt to activate the carbonyl groups. The solution was added dropwise to the chitosan solution, and 1.1522 g (6.01 mmol, 6.0 eq) EDC was further added thereto. Stirring was ended after 18 hours, and dialysis was performed in purified water and sodium hydrogen carbonate. Dialysis was ended after four days, the solution was freeze-dried, and the target chitosan azide was recovered. The ratio of azide groups present was confirmed via ¹H-NMR in a manner similar to that of example 1.

### Example 7

### <B. Synthesizing cyclooctyne group-comprising hyaluronic acid>

### «Adduction of bromoform to cycloheptene»

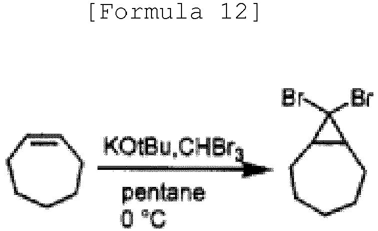

100 mL dehydrated pentane was poured into a three-mouthed flask, 7.62 g (79.27 mmol) cycloheptene and 17.77 g (0.16 mol) potassium-tert-butoxide were added thereto, and the whole was stirred in an ice bath in a nitrogen atmosphere. After 40 minutes, 30.02 g (0.12 mol) bromoform was added dropwise using a microsyringe. After adding dropwise over 30 hours, the whole was stirred overnight at room temperature. Next, 200 mL purified water and roughly 2 mL 12 M HCl were added thereto to neutralize the solution (pH 10.8 to roughly 6). The obtained liquid was poured into an extraction funnel, and the organic phase was recovered. 100 mL pentane was added to the remaining aqueous phase, and extraction was performed (repeated three times). The recovered organic phase was again poured into an extraction funnel, and washed with 200 mL purified water added thereto (repeated three times). Magnesium sulfate was added to roughly 500 mL of the removed organic phase and the whole was dried overnight, after which the magnesium sulfate was filtered out, and the solvent was distilled out via evaporation. Vacuum drying was performed to obtain a dark brown target product.

### «Glycol methyl adduction»

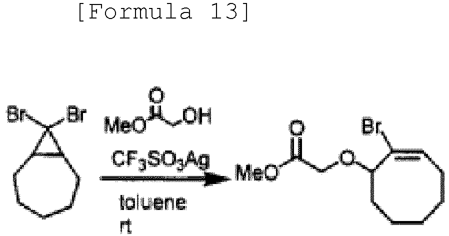

After adding 4.01 g (14.96 mmol) of the dibromide obtained and 13.37 g (0.15 mol) methyl glycolate to an eggplant-shaped flask, 20 mL toluene was added to create a solution. Aluminum foil was used to block light out of the flask, after which 11.43 g (44.49 mmol) silver trifluorosulfonate was added and the whole was stirred as-is for 28 hours at room temperature, after which the toluene was distilled away using an evaporator. Flash column chromatography (eluate (hexane: ethyl acetate = 5:1)) was performed (Rf value: 3.5-4). After passing roughly 2.5 times the column volume of eluate through the column, trans body elution was confirmed. Subsequent solution was recovered, and elution was continued until roughly 10 times the column volume. The eluate was returned to the eggplant-shaped flask, and the solvent was distilled away using an evaporator. Subsequently, vacuum drying was performed to obtain a deep yellow target product (yield: 2.84 g (10.23 mmol); yield rate: 68.4%).

### «Converting to debrominated alkyne and hydrolyzing terminal methyl esters»

HBr was removed from the substance obtained above via E2 elimination to yield an alkyne, after which terminal methyl ester groups were hydrolyzed to obtain carboxylic acid.

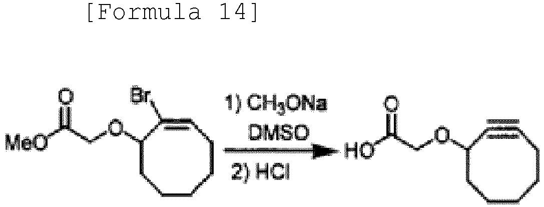

3.76 g (69.56 mmol) sodium methoxide was added to an eggplant-shaped flask and dissolved in 110 mL dehydrated methanol, 6.5 mL dehydrated DMSO was added dropwise thereto, and the whole was stirred. After ten minutes, 2.84 g (10.23 mmol) of the cyclooctyne derivative obtained above was added dropwise and the whole was stirred overnight in a nitrogen atmosphere at room temperature, after which the methanol was distilled away using an evaporator. After adding the obtained solution to an extraction funnel, 200 mL 1.2 M HCl and 100 mL methyl chloride were added, and extraction was performed. 100 mL methylene chloride was added to the remaining aqueous phase, and extraction was performed (repeated three times). The organic phase was recovered, magnesium sulfate was added and the whole was dried overnight, after which the magnesium sulfate was filtered out and the solvent was distilled away using an evaporator. Subsequently, vacuum drying was performed to obtain a deep yellow target product (yield: 2.2485 g (12.34 mmol); yield rate: 121%).

### «Synthesizing cyclooctyne group-comprising hyaluronic acid»

Hyaluronic acid AGUs, carbonyl imidazole (CDI), and the cyclooctyne group-comprising carboxylic acid obtained above were used at theoretical ratios of 1:2:2.

Hyaluronic acid (Mw = 80,000 Da) was added to an eggplant-shaped flask and dissolved in DMSO.

The obtained cyclooctyne group-comprising carboxylic acid and CDI were added to a separate eggplant-shaped flask and dissolved in dimethyl sulfoxide (DMSO), the carboxyl groups of the obtained cyclooctyne group-comprising carboxylic acid were activated, and the whole was reacted for one hour.

The reaction product was added dropwise to the DMSO solution of hyaluronic acid. The reaction product was heated to 60°C starting 10 minutes prior to being added dropwise, and, after 16-20 hours, heating and stirring was completed, and the whole was left to stand at room temperature. One hour after being left to stand, the whole was dialyzed in 3 L purified water, the water being changed out every hour. After continuing dialysis for three days, the whole was recovered in a falcon tube and freeze-dried to obtain a cyclooctyne group-comprising hyaluronic acid B-1.

The presence of 25.3 mol% cyclooctyne groups was confirmed via ¹H-NMR from the ratio of protons derived from the hyaluronic acid (Mw = 80,000 Da) used and protons derived from the cyclooctyne groups constituting the side chain, taking the total amount of -OH groups in the hyaluronic acid (Mw = 80,000 Da) as 100 mol%.

### Example 8

### <<Synthesizing cyclooctyne group-comprising hyaluronic acid B-2>>

An cyclooctyne group-comprising hyaluronic acid B-2 was obtained according to a method similar to that of example 6, except that hyaluronic acid (Mw = 800,000 Da) was used instead of the hyaluronic acid (Mw = 80,000 Da) of example 6. The presence of 14.6 mol% cyclooctyne groups was confirmed via ¹H-NMR in a manner similar to that of example 6.

### Example 9

### <<Synthesizing cyclooctyne group-comprising carboxymethyl dextran B-3>>

Cyclooctyne group-comprising carboxymethyl dextran B-3 was obtained in a manner similar to that of example 6, except that carboxymethyl dextran (Mw = 40,000 Da) was used at an AGU:CDI:gamma-butyric acid azide theoretical ratio of 1:3:3 instead of the hyaluronic acid (Mw = 80,000 Da) of example 6. The presence of 56.6 mol% cyclooctyne groups was confirmed via ¹H-NMR in a manner similar to that of example 6.

### Example 10

### <<Synthesizing cyclooctyne group-comprising carboxymethyl dextran B-4>>

Cyclooctyne group-comprising carboxymethyl dextran B-4 was obtained in a manner similar to that of example 8, except that carboxymethyl dextran (Mw = 160,000 Da) was used instead of the carboxymethyl dextran (Mw = 40,000 Da) of example 8. The presence of 12.4 mol% cyclooctyne groups was confirmed via ¹H-NMR in a manner similar to that of example 6.

### Example 11

### <<Synthesizing cyclooctyne group-comprising carboxymethyl dextran B-5>>

Cyclooctyne group-comprising carboxymethyl dextran B-4 was obtained in a manner similar to that of example 8, except that carboxymethyl dextran (Mw = 580,000 Da) was used instead of the carboxymethyl dextran (Mw = 40,000 Da) of example 8. The presence of 25.3 mol% cyclooctyne groups was confirmed via ¹H-NMR in a manner similar to that of example 6.

### Example 12

### <<Synthesizing cyclooctyne group-comprising chitosan B-6>>

Cyclooctyne group-comprising chitosan B-6 was obtained in a manner similar to that of example 8, except that chitosan was used instead of the carboxymethyl dextran (Mw = 40,000 Da) of example 8. The ratio of cyclooctyne groups present was confirmed via ¹H-NMR in a manner similar to that of example 6.

### Example 13

### <Preparing hydrogels>

### «Preparing polymer solutions»

The azide group-comprising hyaluronic acid A-1 was dissolved in phosphate buffered saline (PBS) to prepare PBS azide group-comprising hyaluronic acid solutions X-1 to X-5 having respective concentrations of 5.0, 3.0, 2.0, 1.5, and 1.0 wt%.
PBS solutions Y-1 to Y-5 of cyclooctyne group-comprising hyaluronic acid B-1 having respective concentrations of 5.0, 3.0, 2.0, 1.5, and 1.0 wt% were similarly prepared.

### «Preparing hydrogels»

100 µL of PBS solution X-1 was added dropwise to a 35 mm Iwaki dish, after which 100 µL PBS solution Y-1 was added dropwise, and a hydrogel Z-1 was obtained at a measured time of eight minute, nineteen seconds from dropwise addition until gelling.

Hydrogels Z-2 to Z-4 were similarly obtained using PBS solutions X-2 to X-4 and PBS solutions Y-2 to Y-4.

No gelling was confirmed from the combination of PBS solution X-5 and PBS solution Y-5. Results are shown in table 1.

[Table 1]

**Table 1. Solutions for preparing hydrogels and gelling times**

| Azide group side | Cyclooctyne group side | Hydrogel | Gelling time |
|---|---|---|---|
| X-1 | Y-1 | Z-1 | Roughly 8 minutes |
| X- | Y-2 | Z-2 | Roughly 24 minutes |
| X-3 | Y-3 | Z-3 | Roughly 43 minutes |
| X-4 | Y-4 | Z-4 | Roughly 1 hour, 20, minutes |
| X-5 | Y-5 | No gelling | - |

### Example 14

### <Preparing hyaluronic acid hydrogels>

### <<Preparing polymer solutions>>

PBS solutions X-6 to X-10 of azide group-comprising hyaluronic acid A-2 and PBS solutions Y-6 to Y-10 of cyclooctyne group-comprising hyaluronic acid B-2 were prepared in a manner similar to that of example 11, except that azide group-comprising hyaluronic acid A-2 was used instead of azide group-comprising hyaluronic acid A-2 and cyclooctyne group-comprising hyaluronic acid B-2 was used instead of cyclooctyne group-comprising hyaluronic acid B-1.

### <<Preparing gels>>

Gels Z-6 to Z-10 were obtained from PBS solutions X-6 to X-10 and PBS solutions Y-6 to Y-10 according to a method similar to that of example 11.

Results are shown in table 2.

[Table 2]

**Table 2. Solutions for preparing gels, gelling, and gelling time**

| Azide group side | Cyclooctyne group side | Gel | Gelling time |
|---|---|---|---|
| X-6 (5.0 wt%) | Y-6 (5.0 wt%) | Z-6 | Roughly 7 minutes |
| X-7 (3.0 wt%) | Y-7 (3.0 wt%) | Z-7 | Roughly 17 minutes |
| X-8 (2.0 wt%) | Y-8 (2.0 wt%) | Z-8 | Roughly 34 minutes |
| X-9 (1.5 wt%) | Y-9 (1.5 wt%) | Z-9 | Roughly 52 minutes |
| X-10 (1.0 wt%) | Y-10 (1.0 wt%) | Z-10 | Roughly 1 hour, 40 minutes |

It is apparent from tables 1 and 2 that gelling time varies according to the molecular weight of the hyaluronic acid and the concentration of the PBS solution used.

For hydrogel swelling, taking initial weight as 100%, volume swelled in a range from 100% to 400% of the hyaluronic acid, breakdown occurred over at least 250 hours in the PBS, and breakdown and elimination occurred in roughly 70 hours in DMEM medium containing 10% bovine serum.

### Example 15

### <Preparing carboxymethyl dextran hydrogels>

A hydrogel was prepared using azide group-comprising carboxymethyl dextran A-4 and cyclooctyne group-comprising carboxymethyl dextran B-4.

Specifically, a PBS solution X-11 (concentration: 5.0 wt%) of azide group-comprising carboxymethyl dextran A-4 and a PBS solution Y-11 (concentration: 5.0 wt%) of cyclooctyne group-comprising carboxymethyl dextran B-4 were prepared in a manner similar to example 11, except that azide group-comprising carboxymethyl dextran A-4 was used instead of azide group-comprising hyaluronic acid A-1, and cyclooctyne group-comprising carboxymethyl dextran B-4 was used instead of cyclooctyne group-comprising hyaluronic acid B-1.

The two halves of the double syringe shown in FIG. 2 were filled using solutions X-11 and Y-11, respectively, and added dropwise to a 35 mm Iwaki dish to obtain a hydrogel Z-11.

### Example 16

A hydrogel was prepared using azide group-comprising chitosan A-6 and cyclooctyne group-comprising chitosan B-6.

Specifically, a PBS solution X-11 (concentration: 5.0 wt%) of azide group-comprising chitosan A-6 and a PBS solution Y-11 (concentration: 5.0 wt%) of cyclooctyne group-comprising chitosan B-4 were prepared in a manner similar to example 11, except that azide group-comprising chitosan A-6 was used instead of azide group-comprising hyaluronic acid A-1, and cyclooctyne group-comprising chitosan B-6 was used instead of cyclooctyne group-comprising hyaluronic acid B-1.

The two halves of the double syringe shown in FIG. 2 were filled using solutions X-11 and Y-11, respectively, and added dropwise to a 35 mm Iwaki dish to obtain a hydrogel Z-12.

### Example 17

### <Hydrogel biocompatibility>

The cytotoxicity of the hydrogels was evaluated using a LIVE/DEAD Assay (Lonza, Inc.). NIH 3T3 NIH Swiss mouse fetal fibroblasts and a D-MEM (prepared) medium were used.

### «Preparing polymer solutions for preparing hydrogels»

The hyaluronic acid A-1 obtained in example 1 was UV sterilized and dissolved in PBS to obtain a PBS solution X'-1 (concentration: 5.0 wt%) of hyaluronic acid A-1.

Similarly, the hyaluronic acid B-1 obtained in example 6 was UV sterilized and dissolved in PBS to obtain a PBS solution Y'-1 (concentration: 5.0 wt%) of hyaluronic acid B-1.

### «Preparing cellular suspensions»

10 mL PBS was added to a 150 cm³ flask in which NIH3T3 cells had been cultured, and washing and suctioning were performed to remove free cells and dead cells. 5 mL trypsin was added to the same flask, which was incubated for five minutes in a 37°C constant temperature bath to debond the cells.

20 mL medium was added to the flask, and the debonded cells were recovered in a 50 mL falcon tube along with the trypsin. A part thereof was portioned out and stained with trypan blue, and the number of cells was counted.

The falcon tube was centrifuged at 1,000 rpm for three minutes to collect the cells at the bottom of the tube.

The supernatant was removed using an aspirator, PBS was added thereto, and a liquid cellular suspension having a cell concentration of 5×10⁶/mL was prepared so as to have a uniform cell distribution.

The cellular suspension obtained above was added to the polymer solutions X'-1 and Y'-1 prepared above to prepare PBS solutions X"-1 and Y"-1 having a polymer concentration of 3.0 wt% and a cell concentration of 2×10⁶/mL.

The obtained PBS solutions X"-1 and Y"-1 were used to fill the two halves of a double syringe (Baxter, Ltd.), and dispensed dropwise into a 35 mm glass-bottomed Matsunami dish while being mixed. The mixture was left standing for two hours at room temperature to obtain a hydrogel Z-14.

4 mL fresh medium was then added to the dish, and incubation was performed in a 37°C constant temperature bath. After two days, the medium was suctioned away using an aspirator, the gel remaining in the dish was immersed in PBS, and any medium suffusing the gel Z-14 was removed. After confirming that all medium had been removed from the hydrogel Z-14, 100 µL each CalceinAM and EthD-1 were added thereto. The whole was left to stand for 30 minutes, and a fluorescent image of the interior of the gel Z-14 was acquired using a confocal microscope. The survival rate of cells enclosed within the gel Z-14 was calculated from the ratio of the numbers of cells stained using the different pigments. Specifically, image enhancement, noise removal, object separation, and manual correction were performed, and cells having a constant fluorescent intensity and fluorescence radius were counted. The ratio of green fluorescent intensity, indicating living cells, and red fluorescent intensity, indicating dead cells, was considered the survival rate. Even counting cells having both types of fluorescence overlapping as dead cells, the survival rate was 68% after one day and 64% after two days. From these results, it was confirmed that the hydrogel Z-14 was biocompatible.

### Example 18

### <In vivo hydrogel preparation>

Living rats were hypodermically injected with a hyaluronic acid-based polymer solution to form a hydrogel *in situ* within the body, and the effects upon surrounding tissues and metabolic organs were histologically evaluated.

### «Preparing polymer solutions for preparing hydrogels»

The hyaluronic acid A-1 obtained in example 1 was sterilized for three hours via UV sterilization and dissolved in PBS to obtain a PBS solution X'-3 (concentration: 2.0 wt%) of hyaluronic acid A-1.

Similarly, the hyaluronic acid B-1 obtained in example 6 was UV sterilized and dissolved in PBS to obtain a PBS solution Y'-3 (concentration: 2.0 wt%) of hyaluronic acid B-1.

### <<In vivo hydrogel preparation>>

The obtained PBS solutions X'-3 and Y'-3 were used to filled the two halves of a double syringe, and a total of 1 mL of the polymer solutions was injected while being mixed into the necks of 9-week-old male SD rates using the double syringe. No signs of acute toxicity were observed in the rats following injection, and health over one week was good.

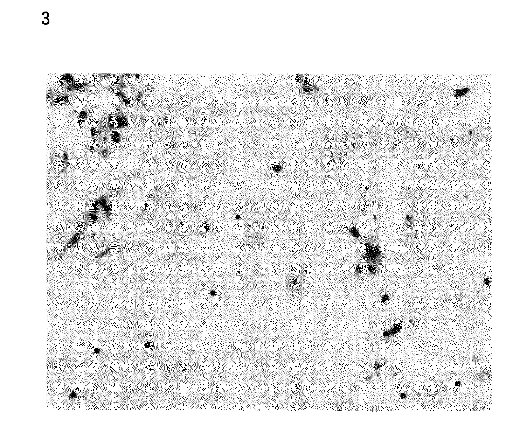

The mice were euthanized after one week, and the organs thereof were observed via dermatotomy and abdominal laparotomy. The tissues surrounding the gel, the liver, the spleen, and the kidneys were excised and preserved in formaline, and tissue slices of the organs were later prepared and stained using hematoxylin and eosin (HE staining). The prepared specimens were observed via optical microscope and histologically evaluated. A stained image of obtained dermal tissue is shown in FIG. 3.

### «Dermatotomy»

The formation of gel within the dermal tissue was confirmed. Thus, it was determined that a hydrogel had been prepared *in vivo* and remained for a maximum of one week.

FIG. 3 shows contamination by a certain level of erythrocytes from bleeding occurring upon injection of the polymer solution, and broad inflammation was confirmed in the vicinity of the gel, but post-HE staining histological evaluation revealed a degree of inflammatory not significantly different from that of foreign body reactions occurring during transplantation of conventional biocompatible biomaterials, and no inflammation was observed within the tissue. This being the case, the gel according to the present invention exhibits a high level of biocompatibility, suggesting the possibility of its use as a novel *in situ* crosslinking gel.

### «Abdominal laparotomy»

### <<<Liver>>>

Discoloration was confirmed on the surface of the liver. Although the cause is not clear, post-HE staining histological evaluation showed that the discoloration was strictly superficial, and no prominent changes were observed in the interior hepatic tissue. There was deformation of the sinusoids near the surface of the liver, indicating a high possibility of the hepatocytes having contracted under some sort of influence. In this test, the polymer solutions were injected hypodermically, and it is believed that degradation products migrated to the blood or lymph and permeated the organs from the interiors thereof, but it is unclear why effects were observed in superficial areas.

### <<<Spleen/kidneys>>>

The spleen produces macrophages, which break down foreign bodies via phagocytosis, and immune antibodies such as B cells and T cells. The kidneys transfer substances having a molecular weight of 50,000 or less dissolved in the blood to the urine via glomerular filtration, expelling them from the body. These organs, like the liver, are those in which the effects of administering materials appear mostly vividly; thus, they were retrieved, HE-stained, and histologically evaluated. The results showed no particular effects in the spleen and kidneys apart from the superficial discoloration of the liver, indicating that they had been kept in a normal state in histopathological terms.

From the foregoing results, it is apparent that no acute toxicity is demonstrated by the PBS solutions X'-3 and Y'-3, as well as the hydrogels formed thereby, when introduced into the body. Moreover, it was shown that the PBS solutions demonstrate *in situ* crosslinking properties.

## Claims

1. A hydrogel comprising:
a) a first polymer section selected from the group consisting of hyaluronic acid, carboxymethyl dextran, cellulose derivatives, and chitosan;
b) a second polymer section, selected from the group consisting of hyaluronic acid, carboxymethyl dextran, cellulose derivatives, and chitosan, that may be the same as or different from a), but comprising a different molecule; and
c) a triazole ring group or derivative group thereof;
the hydrogel having a structure such that the a) first polymer section and b) second polymer section are crosslinked via the mediation of the c) triazole ring group or derivative group thereof.

2. The hydrogel according to claim 1, wherein a spacer group X1 is present between the a) first polymer section and the c) triazole ring group or derivative group thereof, X1 being an alkylene group comprising at least one substituent selected from the group consisting of an ether group, an ester group, an amide group, a hydrazide group, a disulfide group and a carbonyl group.

3. The hydrogel according to claim 1 or 2, wherein a spacer group X2 is present between the b) second polymer section and the c) triazole ring group or derivative group thereof, X2 being an alkylene group comprising at least one substituent selected from the group consisting of an ether group, an ester group, an amide group, a hydrazide group, a disulfide group and a carbonyl group.

4. The hydrogel according to claim 2 or 3, wherein X1 is substituted for an -OH group of the a) first polymer section to create a bond, thereby forming -O-X1-.

5. The hydrogel according to claim 3 or 4, wherein X2 is substituted for an -OH group of the b) second polymer section to create a bond, thereby forming -O-X2-.

6. The hydrogel according to claim 2 or 3, wherein X1 is substituted for an NH₂ group of the a) first polymer section to create a bond, thereby forming -NH-X1-.

7. The hydrogel according to claim 3 or 6, wherein X2 is substituted for an NH₂ group of the b) second polymer section to create a bond, thereby forming -NH-X2-.

8. The hydrogel according to any one of claims 2-7, wherein -X1- is -CO-(CH₂)₃-.

9. The hydrogel according to any one of claims 2-7, wherein -X1- is -CO-NH-(CH₂CH₂O)ₘ-NH-CO-(CH₂)₃- (m representing an integer of 1 or greater).

10. The hydrogel according to any one of claims 3-9, wherein -X2- is -CO-CH₂-O-.

11. The hydrogel according to any one of claims 3-9, wherein -X2- is -CO-NH-(CH₂CH₂O)ₘ-NH-CO-CH₂-O- (*m* representing an integer of 1 or greater).

12. The hydrogel according to any one of claims 1-11, wherein the c) triazole ring group or derivative group thereof is present in an amount equal to 5-60 mol%, taking the total amount of -OH groups or total amount of -NH₂ groups of the a) first polymer section as 100 mol%.

13. The hydrogel according to any one of claims 1-12, wherein the c) triazole ring group or derivative group thereof is present in an amount equal to 5-60 mol%, taking the total amount of -OH groups or total amount of -NH₂ groups of the b) second polymer section as 100 mol%.

14. The hydrogel according to any one of claims 1-13, wherein the a) first polymer section and the b) second polymer section are hyaluronic acid.

15. The hydrogel according to any one of claims 1-13, wherein the a) first polymer section and the b) second polymer section are carboxymethyl dextran.

16. The hydrogel according to any one of claims 1-13, wherein the a) first polymer section and the b) second polymer section are chitosan.

17. A biocompatible material comprising the hydrogel according to any one of claims 1-16.

18. The material according to claim 17, wherein the biocompatible material is an adhesion barrier.

19. The material according to claim 17, wherein the biocompatible material is a drug delivery system carrier.

20. The material according to claim 17, wherein the biocompatible material is a cell- encapsulating material.

21. A kit for creating the hydrogel according to any one of claims 1-16.

22. A method of producing a hydrogel comprising the steps of:
A) readying a liquid containing a) a first polymer selected from the group consisting of hyaluronic acid, carboxymethyl dextran, cellulose derivatives, and chitosan, the first polymer comprising an azide group;
B) readying a liquid comprising b) a second polymer, selected from the group consisting of hyaluronic acid, carboxymethyl dextran, cellulose derivatives, and chitosan, that may be the same as or different from a), but comprising a different molecule than a), the second polymer comprising a cyclooctyne group or a cyclooctyne group derivative; and
C) mixing a) the first liquid and b) the second liquid;
thereby inducing a click reaction between the azide group and the cyclooctyne group or cyclooctyne group derivative, thus forming a triazole ring or derivative thereof, and forming a hydrogel having a structure in which the first polymer and the second polymer are crosslinked via the mediation of the triazole ring or derivative thereof.

23. The production method according to claim 22, wherein step C) is performed *in vivo.*

24. A kit for creating a hydrogel using the production method according to claim 22 or 23.

25. Hyaluronic acid whose -OH groups have been at least partially substituted by a -O-X3-N₃ group, X3 being an alkylene group comprising at least one substituent selected from the group consisting of an ether group, an ester group, an amide group, a hydrazide group, a disulfide group, and a carbonyl group.

26. The hyaluronic acid according to claim 25, wherein -X3- is -CO-(CH₂)₃-.

27. The hyaluronic acid according to claim 25, wherein -X3- is -CO-NH-(CH₂CH₂O)ₘ-NH-CO-(CH₂)₃- (m representing an integer of 1 or greater).

28. The hyaluronic acid according to claim 25 or 26, having the following formula (1) (in which at least one R is a group represented by formula (1)-1, the rest represent H, and n represents an integer from 100 to 20,000.

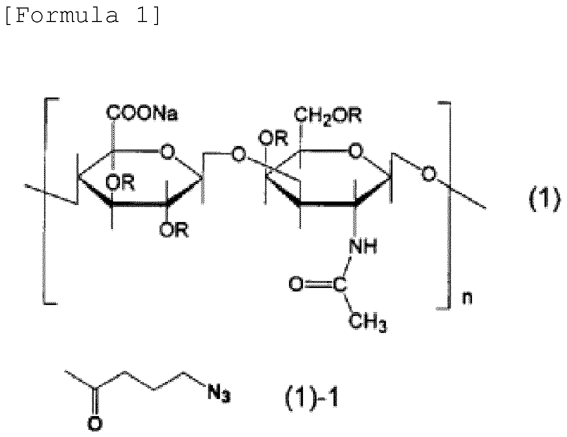

29. Hyaluronic acid whose -OH groups have been at least partially substituted by a -O-X4-(cyclooctyne or cyclooctyne derivative) group, X4 being an alkylene group comprising at least one substituent selected from the group consisting of an ether group, an ester group, an amide group, a hydrazide group, a disulfide group, and a carbonyl group.

30. The hyaluronic acid according to claim 29, wherein -X4- is -CO-CH₂-O-.

31. The hyaluronic acid according to claim 27, wherein -X4- is -CO-NH-(CH₂CH₂O)ₘ-NH-CO-CH₂-O- (m representing an integer of 1 or greater).

32. The hyaluronic acid according to claim 29 or 30, having the following formula (2) (in which at least one R is a group represented by formula (2)-1, the rest represent H, and n' represents an integer from 100 to 20,000.

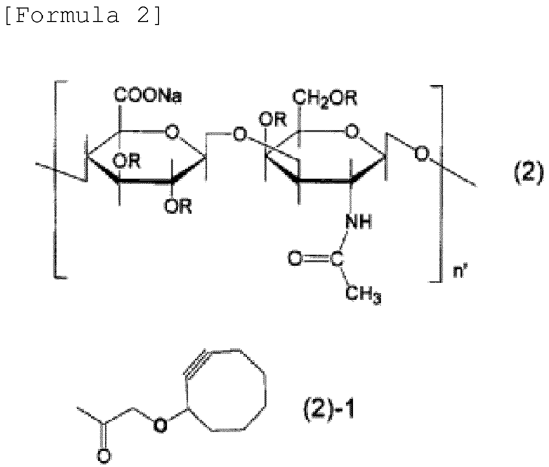

33. Carboxymethyl dextran whose -OH groups have been at least partially substituted by a -O-X3-N₃ group, X3 being an alkylene group comprising at least one substituent selected from the group consisting of an ether group, an ester group, an amide group, a hydrazide group, a disulfide group, and a carbonyl group.

34. The carboxymethyl dextran according to claim 33, wherein the -X3- is -CO-(CH₂)₃-.

35. The carboxymethyl dextran according to claim 33, wherein the -X3- is -CO-NH-(CH₂CH₂O)ₘ-NH-CO-(CH₂)₃- (m representing an integer of 1 or greater).

36. The carboxymethyl dextran according to claim 33 or 34, having the following formula (3) (in which one *R*₁ is CH₂COONa, at least one is a group represented by formula (3)-1, the rest represent H, and i represents an integer from 100 to 20,000).

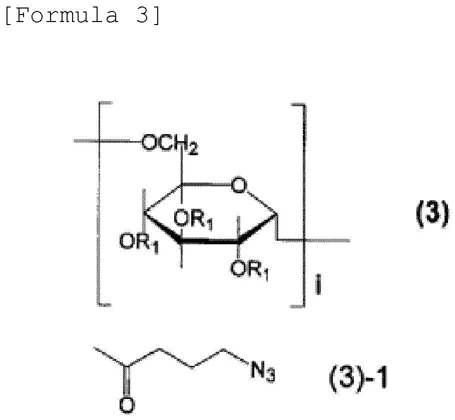

37. Carboxymethyl dextran whose -OH groups have been at least partially substituted by a -O-X4-(cyclooctyne or cyclooctyne derivative) group, X4 being an alkylene group comprising at least one substituent selected from the group consisting of an ether group, an ester group, an amide group, a hydrazide group, a disulfide group, and a carbonyl group.

38. The carboxymethyl dextran according to claim 37, wherein -X4- is -CO-CH₂-O-.

39. The carboxymethyl dextran according to claim 37, wherein -X4- is -CO-NH-(CH₂CH₂O)ₘ-NH-CO-CH₂-O- (m representing an integer of 1 or greater).

40. The carboxymethyl dextran according to claim 37 or 38, having the following formula (4) (in which one *R*₁ is CH₂COONa, at least one is a group represented by formula (4)-1, the rest represent H, and *i*' represents an integer from 100 to 20,000).

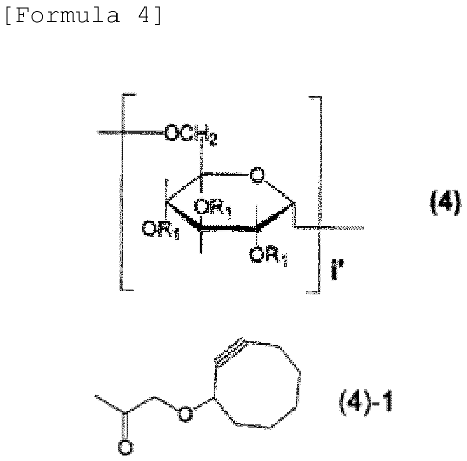

41. Chitosan whose -NH₂ groups have been at least partially substituted by a -NH-X3-N₃ group, X3 being an alkylene group comprising at least one substituent selected from the group consisting of an ether group, an ester group, an amide group, a hydrazide group, a disulfide group, and a carbonyl group.

42. The chitosan according to claim 41, wherein - X3- is -CO-(CH₂)₃-.

43. The chitosan according to claim 41, wherein - X3- is -CO-NH-(CH₂CH₂O)ₘ-NH-CO-(CH₂)₃- (m representing an integer of 1 or greater).

44. Chitosan whose -NH₂ groups have been substituted by a -NH-X4- (cyclooctyne or cyclooctyne derivative) group, X4 being an alkylene group comprising at least one substituent selected from the group consisting of an ether group, an ester group, an amide group, a hydrazide group, a disulfide group, and a carbonyl group.

45. The chitosan according to claim 44, wherein - X4- is -CO-CH₂-O-.

46. The chitosan according to claim 44, wherein - X4- is -CO-NH-(CH₂CH₂O)ₘ-NH-CO-CH₂-O- (m representing an integer of 1 or greater).

47. A method of producing hyaluronic acid whose - OH groups have been at least partially substituted by a-O-X3-N₃ group (X3 being a single bond or a group having a molecular weight of 10,000 or less), the method comprising the steps of:
1) readying hyaluronic acid;
2) readying a substance containing an azide group and a functional group other than an azide group;
3) ion-substituting the hyaluronic acid using a salt comprising a long-chain alkyl ammonium cation that is soluble in both water and an organic solvent, solubilizing the acid in an organic solvent, and preparing a solution of the solubilized acid;
4) obtaining a solution of the substance from 2) in an organic solvent; and
5) mixing the solution from step 4) and the solution of solubilized acid from step 3), and reacting the functional group and the OH groups of the hyaluronic acid via a carboxylation reaction;
thereby obtaining the hyaluronic acid.

48. The method according to claim 47, wherein:
the functional group of the substance from step 2) is a carboxylic acid group; and
the substance is obtained by including steps of:
2)-1) readying a compound having a molecular weight of 10,000 or less, one end of the compound comprising an amino group, and another end comprising a carboxylic acid group; and
2)-2) reacting the compound with an azide compound in a solvent in the presence of a catalyst.

49. A method of producing hyaluronic acid whose-OH groups have been at least partially substituted by a-O-X4-(cyclooctyne or cyclooctyne derivative) group (X4 representing a single bond or a group having a molecular weight of 10,000 or less); the method comprising the steps of:
1) readying hyaluronic acid;
2') readying a substance containing a cyclooctyne group and a functional group other than a cyclooctyne group;
3) ion-substituting the hyaluronic acid using a salt comprising a long-chain alkyl ammonium cation that is soluble in both water and an organic solvent, solubilizing the acid in an organic solvent, and preparing a solution of the solubilized acid;
4) obtaining a solution of the substance from 2) in an organic solvent; and
5) mixing the solution from step 4) and the solution of solubilized acid from step 3), and reacting the functional group and the OH groups of the hyaluronic acid via a carboxylation reaction;
thereby obtaining the hyaluronic acid.

50. The method according to claim 49, wherein:
the functional group of the substance from step 2') is a carboxylic acid group; and
the substance is obtained by including the steps of:
2')-1) reacting a substance comprising a hydroxyl group and an ester group and a bromoform adduct of cycloheptene in an organic solvent in the presence of a catalyst to obtain a substance comprising a 1-bromocyclooctene group and an ester group;
2')-2) converting the substance comprising a 1-bromocyclooctene group and an ester group to a debrominated alkyne in a solvent to obtain a substance comprising a cyclooctyne group and an ester group; and 2')-3) subjecting the substance comprising a cyclooctyne group and an ester group to a hydrolyzing reaction.

51. A method of producing carboxymethyl dextran whose -OH groups have been at least partially substituted by a -O-X3-N₃ group (X3 being a single bond or a group having a molecular weight of 10,000 or less), the method comprising the steps of:
1') readying carboxymethyl dextran;
2) readying a substance containing an azide group and a functional group other than an azide group;
3') ion-substituting the carboxymethyl dextran using a salt comprising a long-chain alkyl ammonium cation that is soluble in both water and an organic solvent, solubilizing the carboxymethyl dextran in an organic solvent, and preparing a solution of the solubilized carboxymethyl dextran;
4) obtaining a solution of the substance from 2) in an organic solvent; and
5') mixing the solution from step 4) and the solution of solubilized acid from step 3), and reacting the functional group and the OH groups of the carboxymethyl dextran via a carboxylation reaction;
thereby obtaining the carboxymethyl dextran.

52. The method according to claim 51, wherein:
the functional group of the substance from step 2) is a carboxylic acid group; and
the substance is obtained by including the steps of:
2)-1) readying a compound having a molecular weight of 10,000 or less, one end of the compound comprising an amino group, and another end comprising a carboxylic acid group; and
2)-2) reacting the compound with an azide compound in a solvent in the presence of a catalyst.

53. A method of producing carboxymethyl dextran whose -OH groups have been at least partially substituted by a -O-X4- (cyclooctyne or cyclooctyne derivative) group (X4 representing a single bond or group having a molecular weight of 10,000 or less); the method comprising the steps of:
1') readying carboxymethyl dextran;
2') readying a substance containing a cyclooctyne group and a functional group other than a cyclooctyne group;
3') ion-substituting the carboxymethyl dextran from step 1') using a salt comprising a long-chain alkyl ammonium cation that is soluble in both water and an organic solvent, solubilizing the carboxymethyl dextran in an organic solvent, and preparing a solution of the solubilized carboxymethyl dextran;
4) obtaining a solution of the substance from 2) in an organic solvent; and
5') mixing the solution from step 4) and the solution of solubilized acid from step 3'), and reacting the functional group and the OH groups of the carboxymethyl dextran via a carboxylation reaction;
thereby obtaining the carboxymethyl dextran.

54. The method according to claim 53, wherein:
the functional group of the substance from step 2') is a carboxylic acid group; and
the substance is obtained by including steps of:
2')-1) reacting a substance comprising a hydroxyl group and an ester group and a bromoform adduct of cycloheptene in an organic solvent in the presence of a catalyst to obtain a substance comprising a 1-bromocyclooctene group and an ester group;
2')-2) converting the substance comprising a 1-bromocyclooctene group and an ester group to a debrominated alkyne in a solvent to obtain a substance comprising a cyclooctyne group and an ester group; and
2')-3) subjecting the substance comprising a cyclooctyne group and an ester group to a hydrolyzing reaction.
